# EUROPEAN PATENT SPECIFICATION

(11) **EP 2 797 413 B1**
(45) Date of publication and mention of the grant of the patent: **12.09.2018**
(21) Application number: 12791835.7
(22) Date of filing: 31.10.2012
(51) Int. Cl.: A01N 25/10, A01N 33/12, C11D 1/62, C11D 3/37, C11D 3/48, C11D 1/72, C11D 1/75, C11D 1/835, C11D 3/43, C11D 3/33

(54) **ANTI-MICROBIAL COMPOSITION**
ANTIMIKROBIELLE ZUBEREITUNG
COMPOSITION ANTIMICROBIELLE

(30) Priority: 29.12.2011 GB 201122407; 29.12.2011 WO PCT/GB2011/052592
(43) Date of publication of application: 05.11.2014
(73) Proprietor: Byotrol PLC, Manchester M40 7RU (GB)
(72) Inventor: HIRST, Rachel, Daresbury WA4 4FS (GB); HURD, Rhiannon Sian, Daresbury WA4 4FS (GB); PLUMMER, Christopher, Daresbury WA4 4FS (GB); MILLS, Timothy Jon, Daresbury WA4 4FS (GB); BURKE, Stephanie, Daresbury WA4 4FS (GB)
(74) Representative: Potter Clarkson LLP
(86) International application number: PCT/GB2012/052710
(87) International publication number: WO 2013/098547

(56) References cited:
- WO-A1-2011/051175
- US-A1- 2003 203 826

## Description

This invention relates to anti-microbial compositions and to formulations including the anti-microbial compositions.

Microorganisms are known to present health hazards due to infection or contamination. They can also cause spoilage of items such as clothing and unpleasant odours. When micro-organisms are present on the surface of a substrate they can replicate rapidly to form colonies.

Many anti-microbial agents that can destroy microorganisms that are present in a wide range of environments such as medical, industrial, commercial, domestic and marine environments are known. Many of the known anti-microbial agents have previously been included in compositions for use in various applications and environments.

The known anti-microbial agents and the compositions that contain these anti-microbial agents destroy microorganisms by a number of different mechanisms.

For example, many anti-microbial agents are poisonous to microorganisms and, therefore, destroy microorganisms with which they are contacted. Examples of this type of anti-microbial agent include hypochlorites (bleaches), phenol and compounds thereof, and salts of copper, tin and arsenic. However, some of these agents can be highly toxic to humans and animals as well as to microorganisms. Consequently these anti-microbial agents are dangerous to handle, and specialist handling, treatment and equipment are therefore required in order to handle them safely. The manufacture and disposal of compositions comprising this type of anti-microbial agent can, therefore, be problematic. There can also be problems associated with the use of compositions containing this type of anti-microbial agent, particularly in consumer materials where it is difficult to ensure that they are used for designated purposes.

Herein, unless the context indicates otherwise, "toxicity" is intended to refer to toxicity to complex organisms such as mammals. References to "toxic" are to be construed accordingly.

Once the anti-microbial agents enter the environment they can affect the health of life forms that they were not intended to affect. Furthermore, the anti-microbial agents are often highly stable and can cause environmental problems for long periods of time.

Other anti-microbial agents currently in use include antibiotic type compounds. Antibiotics disrupt the biochemistry within microorganisms. Although antibiotics are effective, it is believed that they may selectively permit the development of resistant strains of the species that they are used against.

Another method of microbial control is the use of oxidising agents in materials, such as household bleach, which can be based on hypochlorite or peroxides such as hydrogen peroxide. These materials are effective in a wet environment for sterilization and cleansing but stop working shortly after drying.

WO2011/051175 describes a surfactant-containing aqueous anti-microbially effective cleaning agent for hard surfaces, comprising at least one amphoteric polymer, one quaternary ammonium compound, and formic acid, that can be used for cleaning and/or anti-microbially treating hard surfaces, in particular in damp rooms such as bathrooms.

The listing or discussion of an apparently prior-published document in this specification should not necessarily be taken as an acknowledgment that the document is part of the state of the art or is common general knowledge.

There is a need to provide compositions for a variety of applications and uses, such as cleaning applications that have anti-microbia! properties and that address one or more of the problems set out above. However, it is not a straight forward matter to do this. There are regulations such as the Biocidal Products Regulations (Directive 98/8/EC) which regulates the use of anti-microbial agents both in terms of the nature and the amount of a given anti-microbial agent that may be used. Additionally, the potential reactivity of an anti-microbial agent once in a composition is important as some anti-microbial agents are rendered inactive by chemical reaction. Even where an anti-microbial agent is not deactivated by chemical reaction it may have its activity suppressed by other components of the composition.

The present inventors have surprisingly found that the foregoing deficiencies can be overcome by certain combinations of components. It has also been found that compositions containing these combinations of components can have some surprising and unexpected properties.

In particular, the present invention provides anti-microbial compositions suitable for a variety of consumer applications. The compositions of the invention are particularly suitable for use on hard surfaces. Some compositions of the invention are suitable for cleaning a hard surface and also provide a residual anti-microbial effect.

The compositions of the invention can be used for a range of applications and are particularly suitable for use on hard surfaces. The compositions of the invention may be used on hard surfaces indoors such as those found in a domestic setting, an office or a public building such as a hospital or out of doors.

As used herein, by the term cleaning we mean the removal of soils, such as dirt, soap scum and limescale.

The present invention provides an anti-microbial composition which, when subjected to a three wear cycle test on a non-porous stainless steel, glass or plastics substrate provides, a 3 log or greater reduction in micro-organisms over a 24-hour period and comprises (i) an anti-microbial component comprising a quaternary ammonium component (a) comprising at least one quaternary ammonium compound of formula (A) 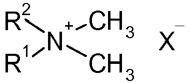 wherein R¹ and R² are each independently a straight chain, unsubstituted and uninterrupted C₈₋₁₂ alkyl group and X⁻ is chloride, bromide, fluoride, iodide, sulphonate, saccharinate, carbonate or bicarbonate and at least one benzalkonium compound of formula (B) 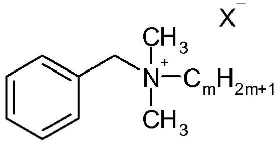 wherein m is from 8 to 18 and X⁻ is chloride, bromide, fluoride, iodide, sulphonate, saccharinate, carbonate or bicarbonate or the benzalkonium compound benzethonium chloride; (ii) a hydrophilic polymer (iii) a polar solvent; (iv) at least one non-ionic surfactant and (v) a chelate; wherein the polymer comprises at least two of the following types of monomer:
(1) a N,N,N-trimethyl-3-((2-methyl-1-oxo-2- propenyl)amino)-1-propanaminium halide monomer or a diallyldimethylammonium halide monomer or a mixture thereof;
(2) an acidic monomer selected from acrylic acid and/or methylacrylic acid; and
(3) a neutral monomer selected from 2-(Dimethylamino)ethyl methacrylate (DMAEMA), N-vinyl pyrrolidone (NVP), N-vinylimidazole, acrylamide, or methacrylamide or a mixture thereof;
and wherein the composition has a pH of 4 or more.

While it is envisaged that compositions of the invention can contain additional ingredients as described below and other ingredients that are standard in the art, the compositions of the invention may consist of or consist essentially of the components listed in the paragraph above.

For the avoidance of doubt, in this specification when we use the term "comprising" or "comprises" we mean that the composition or formulation or component being described must contain the listed ingredient(s) but may optionally contain additional ingredients. When we use the term "consisting essentially of" or "consists essentially of" we mean that the composition or formulation or component being described must contain the listed ingredient(s) and may also contain small (for example up to 5% by weight, or up to 1% or 0.1% by weight) of other ingredients provided that any additional ingredients do not affect the essential properties of the composition, formulation or component. When we use the term "consisting of" we mean that the composition or formulation or component being described must contain the listed ingredient(s) only. These terms can be applied in an analogous manner to processes, methods and uses.

By "substantially free" we mean that the composition or formulation or component being described contains less than 3% by weight, preferably less than 1%, more preferably 0.1% or less by weight of the stated ingredient. For example, the compositions of the invention that are substantially free of alcohol contain less than 3% by weight of alcohol, preferably less than 1% by weight of alcohol, more preferably 0.1% or less alcohol.

By the term "anti-microbial" we mean a compound or composition that kills and/or inhibits the growth of microbes (microorganisms). The term "microbiocidal" is used to refer to compounds or compositions that kill microbes. The compositions of the invention are anti-microbial and/or microbiocidal.

A microorganism or microbe is an organism that is microscopic (too small to be seen by the human eye). Examples of microorganisms include bacteria, fungi, yeasts, moulds, mycobacteria, algae spores, archaea and protists. Microorganisms are generally single-celled or unicellular organisms. However, as used herein, the term "microorganisms" also includes viruses.

The compositions of the invention comprise at least one anti-microbial agent selected from anti-bacterial, anti-fungal, anti-algal, anti-sporal, anti-viral, anti-yeastal and anti-moldal agents and mixtures thereof. More preferably, the compositions of the invention comprise at least one anti-bacterial, anti-viral, anti-fungal and/or anti-moldal agent.

As used herein, the terms anti-bacterial, anti-fungal, anti-algal, anti-viral, anti-yeastal and anti-moldal agents are intended to refer to agents that inhibit the growth of the respective microorganisms but do not necessarily kill the microorganisms and agents that kill the respective microorganisms. Thus, for example, within the term anti-bacterial we include agents that inhibit the growth of bacteria but may not necessarily kill bacteria and bactericidal agents that do kill bacteria.

As the skilled person will appreciate, the word ending "cidal" as used in for example "bactericidal" and "fungicidal" is used to describe agents which kill the microorganism to which it refers. Thus, in these examples, bactericidal refers to an agent that kills bacteria and fungicidal refers to an agent that kills fungus. Examples of bactericides include myobactericides and tuberculocides. Preferably, the compositions of the invention comprise at least one agent selected from bactericidal, fungicidal, algicidal, sporicidal, virucidal, yeasticidal and moldicidal agents and mixtures thereof. More preferably, the compositions of the invention comprise at least one bactericidal, virucidal, fungicidal and/or moldicidal agent.

The compositions of the invention are effective against a wide range of organisms, including Gram negative and Gram positive bacteria, funguses, yeasts, viruses and some spore formers.

The anti-microbial component (i) may comprise (a) at least one, preferably at least two quaternary ammonium compounds having anti-microbial properties and optionally (b) one or more additional anti-microbial agents. Commercially available blends of quaternary ammonium compounds having anti-microbial properties may be used.

The quaternary ammonium anti-microbial agent(s) used in the present invention are typically water soluble at room temperature and pressure.

The anti-microbial quaternary ammonium compounds used in the present invention are compounds of formula (A) 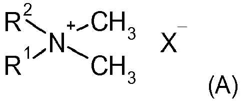 wherein R¹ and R² are each independently a straight chain, unsubstituted and uninterrupted C₈₋₁₂ alkyl group and X⁻ is a halide anion such as chloride, bromide, fluoride, iodide or sulphonate, saccharinate, carbonate or bicarbonate and benzalkonium compounds having the formula (B) 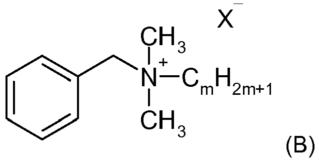 wherein m is from 8 to 18, and X⁻ is a halide anion such as chloride, bromide, fluoride, iodide, sulphonate, saccharinate, carbonate or bicarbonate. The compounds of formula (B) are generally called benzalkonium compounds. The benzalkonium chloride is provided and/or used as a mixture of C₈₋₁₈ alkyl groups, particularly a mixture of straight chain, unsusbtituted and uninterrupted alkyl groups n-C₈H₁₇ to n-C₁₈H₃₇, mainly n-C₁₂H₂₅ (dodecyl), n-C₁₄H₂₉ (tetradecyl), and n-C₁₆H₃₃ (hexadecyl). Preferably m is 8, 10, 12, 14 and/or 16. Most preferably m is from 12 to 16, for example 12, 14 and/or 16.

In the compounds of formula (A) each group R¹ and R² is independently a straight chain, unsubstituted, uninterrupted C₈₋₁₂ alkyl group, for example an alkyl group containing 8, 9, 10, 11 or 12 carbon atoms. In one aspect, the groups R¹ and R² contain the same number of carbon atoms but this is not essential and compounds in which R¹ and R² contain different numbers of carbon atoms can be used.

The anion of each quaternary ammonium compound in the compositions of the invention may be the same or different. For example, the anion of a compound of formula (A) may be the same or different to the anion of a compound of formula (B). In one aspect, the anion for each compound is chloride.

Examples of quaternary ammonium compounds of formula (A) include di-n-decyldimethyl ammonium chloride, octyl decyl dimethyl ammonium chloride and dioctyl dimethyl ammonium chloride.

Examples of commercially available compounds of formula (A) include Acticide® DDQ 50, Bardac® 2250, 2280 and Maquat® 4480E, from Mason Chemical Company, USA.

Examples of quaternary ammonium compounds of formula (B) include N,N-benzyldimethyloctylammonium chloride, N,N-benzyldimethyldecylammonium chloride , N-dodecyl-N-benzyl-N,N-dimethylammonium chloride, N-tetradecyl-N-benzyl-N,N-dimethylammonium chloride, N-hexadecyl-N,N-dimethyl-N-benzylammonium chloride, N,N-dimethyl N-benzyl N-octadecyl ammonium chloride and mixtures thereof.

In one aspect of the invention the quaternary ammonium compound of formula (B) is benzyldimethyl-n-tetradecyl-ammonium chloride, benzyldimethyl-n-dodecyl-ammonium chloride, benzyl-C₁₂-C₁₆-alkyl-dimethyl-ammonium chloride, or benzyl-cocoalkyl-dimethyl-ammonium chloride and/or the compound of formula (A) is di-n-decyldimethyl ammonium chloride, octyl decyl dimethyl ammonium chloride or dioctyl dimethyl ammonium chloride.

Commercially available benzalkonium chloride often contains a mixture of compounds with different alkyl chain lengths. Examples of commercially available benzalkonium chlorides that may be used in the present invention are shown in the following Table. It will be appreciated that other commercially available benzalkonium compounds may alternatively or additionally be used.

| **CAS Number** | **Chemical Name** |
|---|---|
| 63449-41-2 | For example, Acticide Bac 50 - Quaternary ammonium compounds, benzyl C8-18 alkyl dimethyl chlorides (25-50%), from Thor Chemicals in UK, or BQ 451-8 from Albermarle, Global). |
| 68424-85-1 | Alkyl (95% C14, 3% C12, 2% C16) dimethyl benzyl ammonium chloride, for example Barquat MB50/80, both registered as blends using the same CAS number, from Lonza Inc, Switzerland |
| | Alkyl (95% C14, 3% C12, 2% C16) dimethyl benzyl ammonium chloride dehydrate |
| | Alkyl (95% C14, 3% C12, 2% C16) dimethyl benzyl ammonium chloride monohydrate |
| | Alkyl (C14, C12, C16) dimethyl benzyl ammonium chloride |
| | Alkyl dimethyl cumenyl ammonium chloride |
| | Alkyl dimethyl isopropyl benzyl ammonium chloride |
| | Alkyl(68% C12, 32% C14)dimethyl dimethylbenzyl ammonium chloride |
| 8001-54-5 | Alkyl* dimethyl benzyl ammonium chloride *(50% C12, 30% C14, 17% C16, 3% C18), for example NOBAC® BZK, from Mason Chemical Company, USA |

It will be appreciated that a single CAS number often refers to more than one blend or mixture. A CAS classification for a commercial preparation typically covers blends comprising specified compounds in amounts within defined ranges. The compositions having the CAS numbers quoted above are only examples of compositions having a given CAS number that may be used in the present invention.

The amount of component (i) in the compositions of in the present invention will vary depending on a number of factors, such as the intended use of composition and the particular compound(s) used as component (i).

If a mixture of quaternary ammonium compounds of formula A and formula B are used the weight ratio of A to B is not limited and may be from about 1:10 to about 10:1, preferably from 1:5 to 5:1 or from about 1:4 to about 4:1, such as about 2:1 or from about 3:2 to about 2:3.

An example of the quaternary ammonium component is a mixture comprising octyl decyl dimethyl ammonium chloride and/or didecyl dimethyl ammonium chloride and/or dioctyl dimethyl ammonium chloride, and alkyl (C₁₄, 50%; C₁₂, 40%, C₁₆, 10%) dimethyl benzyl ammonium chloride. These compounds may be used together in any suitable ratio. One such ratio is about 2:1:1:2.7 by weight. A suitable mixture is sold commercially as Maquat MQ 615M by Mason Chemical.

As described herein component (i) may consist essentially of or consist of at least one or at least two compounds of formula (A) or may consist essentially of or consist of at least one or at least two compounds of formula (B).

In one aspect, component (i) comprises, consists essentially of or consists of at least one compound of formula (A) and at least one compound of formula (B). In this aspect the ratio by weight of the compound of formula (A) to the compound of formula (B) is for example from 10:1 to 1:10 or 4:1 to 1:4, such as 3:2 or 2:3.

In one aspect, the quaternary ammonium antimicrobial component (i) may consist of (1) a component consisting essentially of a single compound of formula (A); and (2) a component consisting essentially of at least one benzalkonium compound having the formula (B), wherein the ratio by weight of (1) to (2) is from 10:1 to 1:10 or 4:1 to 1:4, such as 3:2 or 2:3.

The quaternary ammonium compounds used in the present invention are typically not polymerized.

The compositions of the invention must contain at least one, such as at least two, quaternary ammonium anti-microbial agents. They may additionally comprise any other suitable anti-microbial agent(s) (b), such as those described in the EPA (United States Environmental Protection Agency) Listing and Annex I and Annex 3 of the EC Biocides Directive.

Suitable anti-microbial agents (b) include anti-microbial agents that are not quaternary ammonium compounds. Preferably, these additional antimicrobial agent(s) are water soluble at room temperature and pressure.

Examples of suitable additional antimicrobial agents include but are not limited to polymeric biguanidines (e.g. polyhexamethylene biguanidine (PHMB)), non-polymeric biguanides (e.g. chlorhexidine), silver, octenidine HCl, amphoteric compounds, iodophores, phenolic compounds, isothiazalones, amine anti-microbial agents and nitrogen based heterocyclic compounds, alkyl betaines, alkyl amine oxides, ortho phenyl phenol (OPP), and nitro bromopropanes (e.g. bronopol (INN), 2-bromo-2-nitropropane-1,3-diol), naturally derived biocidal compounds (e.g. honey and extracts of honey such as those comprising methyl glyoxal, flavenoids based antimicrobials, essential oils and organic acids e.g. lactic acid or citric acid).

In one aspect, the compositions of the invention are free of inorganic antimicrobial agents such as those comprising silver. In this aspect, the additional antimicrobial agent is an organic antimicrobial agent.

Preferred additional antimicrobial agents (b) include polymeric biguanidines. One preferred additional antimicrobial agent (b) is polyhexamethylene biguanidine (PHMB). PHMB is commercially available from Arch Biocides as Vantocil® (industrial grade quality or Cosmocil® (Cosmetic grade quality, For example PHMB20 supplied by Thor).

An example of amine anti-microbial agents that may be used in the compositions of the invention is a compound of formula (C) 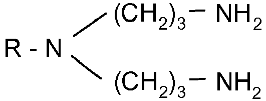 where R is an unsubstituted C₈ to C₁₈ alkyl group. Preferably R is from 10 to 14, for example 12. A preferred compound of formula (C) is dodecyl dipropylene triamine (N,N-bis(3-aminopropyl)-dodecylamine CAS no. 2372-82-9), commercially available from Lonzabac as Lonzabac 12, it is also known as Triameen YD12 (available from Akzo Nobel).

In one aspect, the compositions of the invention do not comprise PHMB or they may be free of polymeric biguanides or they may be free of non-polymeric biguanidines or free of biguanidines (eg free of polymeric and non-polymeric biguanides). In one aspect of the invention the anti-microbial composition does not comprise any isothiazalones and/or any nitrobromopropanes such as bronopol and/or any hypochlorites.

In one particular aspect, compositions of the invention that comprise a compound of formula (C), for example N,N-bis(3-aminopropyl)-dodecylamine do not comprise PHMB or are free of polymeric biguanidines or free of biguanidines (e.g. free of polymeric and non-polymer biguanidines). However, compositions that comprise a compound of formula (C), for example N,N-bis(3-aminopropyl)-dodecylamine and a biguanidine, such as a polymeric or non-polymer biguanidines, for example PHMB are also envisaged.

An example of a composition of the invention is a composition in which component (i) is (a) at least one or at least two quaternary ammonium compounds as described above, for example a compound of formula (A) and a compound of formula (B) and (b) at least one polymeric biguanide such as PHMB, which may be the only additional anti-microbial component (b) or the component (b) may contain a further anti-microbial ingredient.

An example of a composition of the invention is a composition in which component (i) comprises (a) at least one or at least two quaternary ammonium compounds as described above, for example a compound of formula (A) and a compound of formula (B) and (b) at least one compound of formula (C), such as N,N-bis(3-aminopropyl)-dodecylamine.

An example of a composition of the invention is a composition in which component (i) comprises (a) at least one or at least two quaternary ammonium compounds as described above, for example a compound of formula (A) and a compound of formula (B) but does not comprise an additional antimicrobial agent (b).

In one aspect, component (b) does not comprise isothiazole. In this aspect, the compositions of the invention are free of isothiazole.

The compositions of the invention can be provided in concentrated form for dilution before use or in ready to use form. It is preferred that the compositions of the invention are provided in ready to use form and, unless otherwise stated, information about amounts (such as weight% or ppm) provided in this document relate to ready to use compositions.

Typically, but not essentially, the compositions of the invention have a concentration of component (i) of from about 100 or 500 to about 20000 ppm, such as from about 2000 to about 10000 ppm, or 3000 to 8000 ppm.

The polymers suitable for use in the present invention are hydrophilic polymers comprising at least two of the following types of monomer:
(1) a N,N,N-trimethyl-3-((2-methyl-1-oxo-2-propenyl)amino)-1-propanaminium halide monomer or a diallyldimethylammonium halide monomer or a mixture thereof;
(2) an acidic monomer selected from acrylic acid and/or methylacrylic acid; and
(3) a neutral monomer selected from 2-(Dimethylamino)ethyl methacrylate (DMAEMA), N-vinyl pyrrolidone (NVP), N-vinylimidazole, acrylamide, or methacrylamide or a mixture thereof.

Any combination of these types of monomer may be used. For example, suitable polymers include but are not limited to those comprising, consisting of or consisting essentially of at least one monomer of type (1) and at least one monomer of type (2) and polymers comprising, consisting of or consisting essentially of at least one monomer of type (1) and at least one momoner of type (3), polymers comprising, consisting of or consisting essentially of polymers comprising at least one monomer of type (2) and at least one monomer of type (3) and polymers comprising, consisting of or consisting essentially of at least one of each of the three types of monomer.

The polymers used in the present invention may have a polyampholyte structure such that the charge and surface adsorption are determined by pH. For example, the polymer may be an acrylic acid amine-functional polymer. Examples of suitable hydrophilic polymers are described in US6,569,261, US6,593,288, US6,703,358 and US6,767,410, the disclosure of these documents is incorporated herein by reference. These documents describe watersoluble or water-dispersible copolymers including, in the form of polymerized units, (1) at least one amine-functional monomer, (2) at least one hydrophilic monomer with an acidic nature and (3) optionally at least one neutral hydrophilic monomer having an ethylenic unsaturation. The copolymers include quaternized ammonium acrylamide acid copolymers.

Examples of a cationic monomer (1) described herein include but are not limited to: 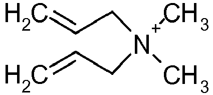 Diallyldimethylammonium halides such as diallyldimethylammonium chloride (DADMAC) or the corresponding bromide. Alternatively, the counter ion may be sulphate or phosphate. Similar momomer units, such as those in which one or more of the CH₃ groups is replaced by a C_{2 to 12} for example a C_{2 to 6} alkyl group or one or more of the CH₂ groups is replaced by an alkyl group having from 2 to 12, for example from 2 to 6 carbon atoms may be used. In other words, other similar commercially available monomers or polymers containing such monomers may be used. 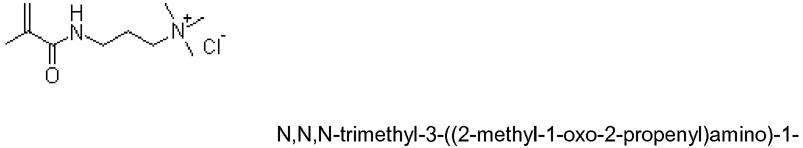 propanaminium halides, such as the chloride (MAPTAC, also known as methacryl-amido(propyl)-trimethyl ammonium chloride).

In the invention, the cationic monomer (1) is a N,N,N-trimethyl-3-((2-methyl-1-oxo-2- propenyl)amino)-1-propanaminium halide monomer or a mixture of a N,N,N-trimethyl-3-((2-methyl-1-oxo-2-propenyl)amino)-1-1propanaminium halide monomer and a diallyldimethylammonium halide monomer.

The acidic monomer (2) is selected from acrylic acid and/or methylacrylic acid.

Examples of neutral monomers (3) described herein include but are not limited to: 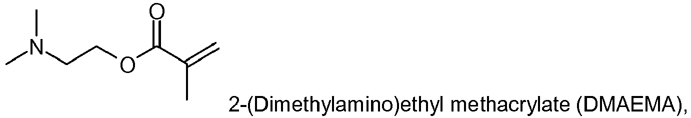 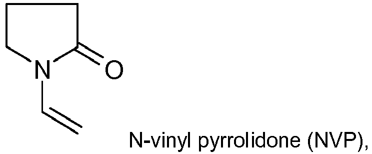 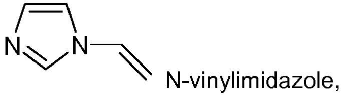 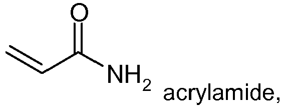 and

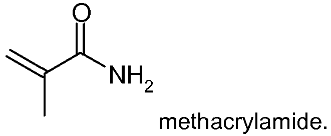

In the invention, the neutral monomer (3) is 2-(Diemthylamino)ethyl methacrylate (DMAEMA), N-vinyl pyrrolidone (NVP), N-vinylimidazole, acrylamide or methacrylamide or a mixture thereof.

An example of a polymer suitable for use in the present invention is a polymer comprising, consisting of or consisting essentially of DMAEMA, MAPTAC and methylacrylic acid.

Suitable polymers include those sold under the trade name Mirapol®®, for example as Mirapol® Surf-S110, Mirapol® Surf-S200 or Mirapol® Surf-S500 available from Rhodia, Novecare.

Other suitable polymers include polymers comprising, consisting of or consisting essentially of DADMAC and acrylamide, such as those sold under the trade name Polyquat 7® or PQ7 from Surfacare or under the trade name Merquat® S from Lubrizol. Other suitable polymers include polymers comprising, consisting of or consisting essentially of DADMAC and methacrylamide and/or, acrylic acid or methacrylic acid.

Polymers comprising, consisting of or consisting essentially of MAPTAC and acrylamide or methacrylamide are also suitable for use in this invention. Also suitable are polymers comprising, consisting of or consisting essentially of MAPTAC and vinyl pyrrolidone, such as Polyquat 28. Suitable polymers include those sold under the trade names Polyquart® Pro. (which is polyquat 28 plus silicone) and Polyquart® Ampo 140 from BASF.

Other suitable polymers include polymers comprising, consisting of or consisting essentially of MAPTAC and acrylic acid or methacrylic acid, such as those sold under the trade name Polyquat® Ampho, eg Polyquat® Ampho 149.

Polymers comprising, consisting of or consisting essentially of DMAEMA and vinylpyrrolidone are suitable for use in this invention. An example of such a polymer is sold under the name PQ11 by BRB International.

Other suitable polymers include polymers comprising, consisting of or consisting essentially of DMAEMA and acrylamide, such as the polymer sold under the trade name Polyquat 5.

Typically the polymers used in the present invention are unmodified. By this we mean that the polymers are, for example, not modified by reactions such as grafting with a functionalizing agent or a hydrophobic agent.

The ratio in terms of parts per million (PPM) of the quaternary ammonium component (a) to the polymer (ii) is typically from 200:1 to 1:20, such as from 1:1 to 20:1 or 10:1, for example about 2:1 or about 4:1. In a particular aspect the amount in ppm of component (a) is greater than or equal to the amount of polymer. For example, the ratio of component (a) to the polymer (ii) may be about 5:1 or about 4:1 or about 3:1 or about 2:1 or about 1:1.

As an example, compositions of the invention may comprise from 500 to 3000 ppm of polymer, such as from 1000 to 2500 ppm or about 2000 ppm.

The compositions of the invention may comprise a single polymer (that is only one combination of monomer units) or two or more polymers may be used (that is polymers made up of two or more combinations of monomer units).

The pH of the compositions of the invention is pH 4 or more or pH 4.5 or more, such as from 5 to 13, for example from about pH 5 to about pH 12, such as from about pH 5 to a pH of 9 or above.

The compositions of the invention comprise at least one suitable non-ionic surfactant or a combination of surfactants including at least one non-ionic surfactant, for example at least one non-ionic surfactant optionally in combination with at least one cationic and/or amphoteric surfactant. The selection of surfactants will depend on the nature of and the intended purpose of the composition. Suitable surfactants for use in formulations intended for different purposes will be within the knowledge of the person of ordinary skill in the art. Likewise, the selection of suitable amounts of surfactant will be within the knowledge of the person of ordinary skill in the art.

Some compositions of the invention comprise an amphoteric surfactant. If a combination of an amphoteric surfactant and a non-ionic surfactant is used the weight ratio of the two types of surfactant can vary within wide limits, for example from 1% of amphoteric surfactant to 99% of non-ionic surfactant to 99% of amphoteric surfactant to 1% of non-ionic surfactant, based on the total weight of surfactant. The amphoteric surfactant and the non-ionic surfactant may, for example, be used in approximately equal amounts by weight.

In one aspect, the composition is substantially free of or free of anionic surfactant. In another aspect, the compositions of the invention do not comprise an amphoteric surfactant.

The compositions of the invention comprise a polar solvent, component (iii). Suitable polar solvents include, but are not limited to, water, alcohols, glycol ethers and mixtures thereof.

Suitable alcohols include, but are not limited to, straight or branched chain C₁ to C₅ alcohols, such as methanol, ethanol, n-propanol, iso-propanol, mixtures of propanol isomers, n-butanol, sec-butanol, tert-butanol, iso-butanol, mixtures of butanol isomers, 2-methyl-1-butanol, n-pentanol, mixtures of pentanol isomers and amyl alcohol (mixture of isomers), and mixtures thereof.

Preferred polar solvents for use in the compositions of the invention include, but are not limited to, water, ethanol, n-propanol, isopropanol, ethylene glycol ethers, propylene glycol ethers, butyl diglycol (BDG) and dipropylene glycol methyl ether (Trade name Dowanol® DPM) and mixtures thereof. In one aspect, the composition comprises water or a mixture of water and one or more alcohols selected from the alcohols described above. In such mixtures, water is preferably the major component. The polar solvent may consist essentially of water or consist of water.

If the compositions of the invention comprise an alcohol, the alcohol is typically present in an amount lower than the amount necessary for the alcohol to provide an antimicrobial effect.

In one aspect, the compositions of the invention are substantially free of alcohol. For example, the compositions may contain 1% or less by weight alcohol. For example, the compositions may contain less than 1% or less than 0.5% by weight or 0.1% by weight or less of an alcohol such as isopropanol. As an example, compositions of the invention may comprise no isopropanol or may comprise no alcohol.

The composition may comprise water or a mixture of water and one or more alcohols selected from the alcohols described above. In such mixtures, water is preferably the major component.

Suitable non-ionic surfactants include, but are not limited to, amine oxides, alkyl polyglucosides, linear and branched 1° and 2° alcohol ethoxylates, nonyl phenol ethoxylates, and ethoxylated/propoxylated (EOPO) block polymers.

In an aspect of the invention, the non-ionic surfactant is an alcohol alkyloxylate or an amine oxide or a mixture thereof.

Amine oxides suitable for use in the present invention are those with an alkyl chain length of C₈-C₁₆. Amine oxides are suitable for use in the present invention because they can provide better cleaning properties and can have less of a degradative effect on surfaces in particular plastic than some other surfactants.

Examples of suitable amine oxides compounds include, but are not limited to, C₈ amine oxide surfactants such as those sold under the trade name Macat® AO-8 (Mason Chemical Company), C₁₀ amine oxides such as those sold under the trade names Euroxide® D40 (EOC Group) and Mackamine® C₁₀ (Rhodia Novecare), Longer chain glucosides such as C₁₂ amine oxides for example those sold under the trade names Euroxide® LO/A (EOC Group), Ammonyx® LO (Stepan Chemicals) and Surfac® AO30 (Surfachem) and d₁₀-C₁₆ amine oxides such as those sold under the trade name Ammonyx® C (Stepan Chemicals).

Alcohol alkoxylates suitable for use in the present invention include primary and secondary linear and branched alcohol ethoxylates, such as C₆-C₁₈ alcohol ethoxylates having 2 to 20 EO units such as 7 to 12 EO units. Preferred primary linear alcohol alkoxylates include C₉₋₁₁ alcohol ethoxylates having 5 to 12 EO units such as 7 to 12 EO units. These surfactants are available under the commercial name of the Neodol® series (From Shell Chemical Company). Examples of secondary C12-15 alcohol ethoxylates, include those that have from about 3 to about 10 EO units, such those available in the Tergitol® series (Dow), particularly those in the Tergitol 15-S series. Examples of suitable branched alcohol exthoxylates include C10 Guerbet alcohol with 3 to 14 EO units such as Lutensol® XL and XP series (available from BASF). Alcohol ethoxylates having EO and PO mixtures are also suitable, these include
linear and branched alcohol ethoxylates with PO-EO units such as ECOSURF® EH Surfactants (DOW) and Plurafac® D250 (BASF). Alkylphenol ethoxylate (APE) surfactants may also be used.

A further class of non-ionic surfactants that may be used is alkoxy block copolymers, in particular, compounds based on ethoxy/propoxyl block copolymers. Polymeric alkylene oxide block copolymers include non-ionic surfactants in which the major portion is made up of block polymeric C₂₋₄ alkylene oxides. Specific examples include surfactants commercially available under the Pluronic® trade name and in particular the Pluronic F series, Pluronic L series, Pluronic P series and Pluronic R series.

A further class of suitable non-ionic surfactants are alkyl polyglucosides or glucosides. Suitable examples include Surface APG a capryl glucoside available from Surfachem and the Glucopon® seriers available from BASF (Glucopon 425N/HH (C8-14), Glucopon 215 (C8-10) and Glucopon 600 (C12-14) alkyl polyglucoside).

In one aspect of the invention, the compositions of the invention may be free of alcohol alkoxylate. For example, compositions of the invention that contain a compound of formula (C) as defined herein such as those containing N,N-bis(3-aminopropyl)-dodecylamine may be free of alcohol alkoxylate.

The compositions of the invention may alternatively or additionally be free of alkyl polyglucoside. For example, compositions of the invention that contain a compound of formula (C) as defined herein such as those containing N,N-bis(3-aminopropyl)-dodecylamine may be free of alkyl polyglucoside (APG).

Typically, the component (iv), is present in the compositions of the invention in an amount of from about 500 to 35000 ppm. The amount of surfactant (iv) depends on a number of factors such as the pH of the composition and the intended use of the composition. Typically higher pH compositions require higher concentrations of non-ionic surfactant. A preferred range for the amount of surfactant (iv) for a composition having a pH of from 8 to 10.5 is from 2000 to 13500 ppm.

Suitable amphoteric surfactants include but are not limited to C₆-C₂₀ alkylamphoacetates or amphodiacetates (such as cocoamphoacetates), C₁₀-C₁₈ alkyldimethyl betaines, C₁₀-C₁₈ alkyl amidopropyldimethyl betaines. Examples include but are not limited to coconut amphoteric surfactant cocoamidopropyl betaine (CAPB) (Surfac® B4, CAS 61789-40-9), coco imidazoline betaine, oleo amido propyl betaine, and tall oil imidazoline.

Without wishing to be bound by theory, it has been found that the surfactant component can have several effects on the properties of the compositions of the invention. The presence of a non-ionic surfactant can improve the cleaning ability of the composition, that is improve the removal of soils and dirt. It has been found that the use of an alcohol alkoxylate in combination with an amine oxide can in some situations improve cleaning performance. Additionally, the inclusion of non-ionic surfactants can improve the stability of the compositions of the invention.

The present inventors have surprisingly found that by using the combinations described herein stable compositions that provide residual antimicrobial performance can be obtained.

In one aspect, the compositions of the invention are free of glycol ethers, for example they are free of propylene glycol n-butyl ether and/or free of a binary solvent combination of a glycol with a linear primary alcohol.

The compositions of the invention comprise a chelate. Any suitable chelate may be used. Suitable chelates include but are not limited to EDTA (Ethylenediaminetetraacetic acid), Gluconate, GLDA (Glutamic acid diacetic acid) - Trade name Dissolvine GL, EDDS (Ethylenediamine-N,N'-disuccinic acid), DPTA (Diethylenetriaminepentaacetic acid), HEDTA (Hydroxyethylethylenediaminetriacetic acid), MGDA (Methyl glycine diacetic acid) - Trade name Trilon M, PDTA (1,3-propylenediaminetetraacetic acid), and EDG (Ethanoldiglycineic acid) and mixtures thereof. If the chelate contains a counter ion that counter ion is preferably metallic. Suitable metallic counter ions include but are not limited to Na, Ca, Fe, K, Zn, Mg and Mn.

Preferred chelates are GLDA (Dissolvine) and EDTA.

The chelate is typically present in an amount of from about 100 to 10,000 ppm, preferably from about 400 to 3,000 ppm, for example from about 500 to 2000ppm.

It will be appreciated that the compositions of the invention can comprise other ingredients commonly used in the art. The nature of any other ingredients used will depend on the nature and intended purpose of the composition. The person of ordinary skill in the art will know which additional ingredients are suitable for use in compositions for different applications.

Additional ingredients that may be used in the compositions of the invention include but are not limited to buffering agents include, but are not limited to, hydrophobic materials such as siloxanes, bicarbonate salts such as sodium bicarbonate, sodium chloride, potassium chloride, triethylamine, triethylenetetramine tetraethylethylenediamine, tetramethylenediamine, piperazines, histadines, imidazoles, morpholines, aminoalcohols such as 2-aminoethanol and 2-amino-2-methyl-1-propanol (AMP) and triethanolamine, phosphate buffers, citrate buffers and salts thereof and mixtures thereof.

Complexing agents may be used. For example, complexing agents can be used to help provide a clear composition even when the compositions of the invention are used with hard water. Suitable complexing agents include but are not limited to sodium salts of methane diphosphonic acid, hydroxythenae-1,1-diphosphonic aicd, 1-aminoethane-1,1-diphosphonic acid, amino -trimethylene phosphonic acid, ethylene diamine-tetra (methylene phosphonic acid), diethylene triamine-penta (methylene phosphonic acid), 2-phos-phonobutane-1,2,4-tricarboxylic acid, and nitrilotriacetic acid (NTA), citrates and gluconates or salts of glutaric, adipic and succinic acids and mixtures thereof.

pH modifiers may be used. Suitable pH modifiers include but are not limited to acids such as citric, sulfamic, hydrochloric, phosphoric, nitric, lactic, formic, acetic glycolic or gluconic acids or other mineral or organic acids or bases such as sodium or potassium hydroxide and carbonates and mixtures thereof. The compositions of the invention may alternatively or additionally contain salts such as the halides of alkali metals or alkaline earth metals such NaCl or KCl. In some situations, the use of salts can facilitate the formation of a stable composition.

Some compositions of the invention are free of or substantially free of acids or bases. For example some compositions of the invention do not comprise a citrate or citric acid or lactic acid. Other compositions may contain an acid or base as is appropriate for their intended use. In one aspect of the invention the composition contains 0.5% by weight or less of formic acid or is free of formic acid.

Siloxanes suitable for use in the present invention include those having formulae (H₃C)[SiO(CH₃)₂]ₙSi(CH₃)₃, and (H₃C)[SiO(CH₃)H]ₙSi(CH₃)₃, and mixtures thereof, where n is an integer, of from 1 to 10, more preferably from 1 to 6 and most preferably from 1 to 4, for example n may be 1, 2, 3, 4, 5, 6, 7, 8, 9, 10, especially 1, 2, 3 or 4. Examples of preferred (poly)dimethylsiloxanes are hexamethyldisiloxane (CAS # 107-46-0), octamethyltrisiloxane (CAS # 107-51-7), decamethyltetrasiloxane (CAS # 141-62-8), dodecamethylpentasiloxane (CAS # 141-63-9). Commercially available siloxanes that may be used include those available from Xiameter under the trade names PMX 200 and PMX-1184.

In a particular aspect of the invention the compositions of the invention do not comprise or are substantially free of a hydrophobic material, for example they do not comprise or are substantially free of siloxanes, silicones, polysiloxanes such as polydimethylsiloxanes.

The compositions of the invention may also contain other ingredients that are standard in the art such as colorants, fragrances, emollients, antioxidants, thickeners and corrosion inhibitors and mixtures thereof.

Typically, component (i) is present in the compositions of the invention in an amount of from about 0.05 to about 2 % by weight of the compositions, such as from about 0.2 or about 0.5 to about 1.5 or about 1.0 %.

The ratio by weight of the component (a) to the additional anti-microbial agent (b) is typically from about 1:10 or about 1:2 or about 1:1 to about 50:1, or to about 30:1, for example, from about 1:1 or about 2:1 or about 3:1 to about 20:1 or about 10:1 or about 6:1 or from about 0.4:1 to about 4:1, such as about 0.55:1 to about 3.16:1, for example about 1:1, or about 2:1, or about 2.75:1 or about 3:1 or 4:1.

Typically, the polar solvent component (iii) is present in the compositions of the invention in an amount of from about 10 to about 99.999% by weight of the compositions, such as from about 50 to about 99.999%, for example from about 80 to about 99.99%, preferably from about 90 to about 99.9%, more preferably from about 95 to about 99.8% (e.g. from 97 to 99.7% or 97.5 to 99.6%).

Typically, the additional anti-microbial agent component (b) is present in the compositions in an amount of from about 0.001 to about 10% by weight of the compositions, such as from about 0.005 to about 5%, for example from about 0.01 to about 2%, preferably from about 0.05 to about 1% (e.g. from 0.1 to 0.5%).

An example of a composition of the invention is a composition comprising:
(i) (C₁₀H₂₁)₂(CH₃)₂N⁺Cl⁻ and a benzalkonium chloride, which may be in a ratio in terms weight from about 1:10 to 10:1, for example about 4:1;
(ii) a polymer as defined above, such as a polymer comprising DMAEMA, MAPTAC and methylacrylic acid, or DADMAC and acrylamide, methacrylamide, acrylic acid or methacrylic acid, or MAPTAC and acrylamide or methacrylamide, or MAPTAC or DMAEMA and vinyl pyrrolidone, or MAPTAC and acrylic acid or methacrylic acid, or DMAEMA and acrylamide;
(iii) water;
(iv) a non-ionic surfactant such as an alcohol ethoxylate or amine oxide or a mixture thereof; and
(v) a chelate such as EDTA or GLDA.
This composition has a pH of at least 2.5, such as at least 3.5.

It is preferred that the polymer comprises DMAEMA, MAPTA and methylacrylic acid, a commercial example of which is sold under the trade name Mirapol® Surf-S 110.

Without wishing to be bound by theory, the inventors have found that there are very significant advantages associated with the compositions of the invention.

It has been found that in use compositions of the invention have advantageous anti-microbial effects. For example, such compositions have an anti-microbial effect when initially applied to a surface (so called "wet kill") and they can also have a residual anti-microbial effect in that they control, reduce or prevent the formation of new microbial colonies at the surface (so called "dry kill").

The compositions of the invention are also resistant to washing with water and to wiping. This means that the compositions of the invention provide a residual anti-microbial effect even when the surface which has been treated is subsequently wiped and/or washed or rinsed with water.

The compositions of the invention provide a residual antimicrobial effect and provide an anti-microbial effect even after being subjected to the residual efficacy test described herein. That is, these compositions provide a reduction in micro-organisms when subjected to a three wear cycle test on a non-porous stainless steel, glass or plastics substrate and typically provide a 3 log or greater reduction over a 24 hour period. By this we mean that 24 hours after application of the composition of the invention to the surface, if micro-organisms are applied to that surface (without the further addition of a composition of the invention or another anti-microbial agent) at least a 3 log reduction in those micro-organisms will be achieved.

An advantage of the invention is that it is possible to prevent a broad range of microorganisms from adhering and attaching to the surface, and, therefore, from forming a biofilm. Large numerous colonies are also substantially prevented from forming. Thus, the ability of the colony to grow is substantially reduced or even prevented. The invention is therefore general in its control of microorganisms.

Typically, the compositions of the invention do not need to contain materials that are highly toxic to mammals. The anti-microbial agents used in the anti-microbial compositions are typically well known and widely understood and tested anti-microbial agents. The efficacy of the known anti-microbial agents is amplified in the formulations of the invention. Therefore, anti-microbial agents that have a low toxicity can be used in the anti-microbial compositions. In contrast, many "new" anti-microbial agents for known techniques of sanitization use "stronger", more toxic and/or little tested materials.

The anti-microbial compositions of the invention do not contain materials that produce highly persistent residues or rinsates or products that contain heavy metals and their salts. Thus, there is a greatly reduced risk of long term hazards.

The anti-microbial compositions of the invention do not interfere with the biochemical reproductive pathways of the micro-organisms they control. The risk of resistance build up and the development of resistant strains is, therefore, low.

The anti-microbial compositions of the invention can have a duel effect in that not only do they provide an anti-microbial effect in use but they can also have a preservative effect on the composition. This means that it is typically not necessary to include additional preservatives in the formulations of the invention.

The compositions of the invention do not typically give surfaces to which they are applied a greasy feel.

According to a further aspect of the invention, there is provided the use of composition of the invention to control, reduce or prevent the formation of colonies of micro-organisms on a surface at which it is provided.

The compositions of the invention can be used in a method for providing a residual antimicrobial benefit to a surface such as a hard surface or skin, which method comprises applying a composition as defined herein to that surface. The composition may be applied to the surface by spraying the composition on the surface or wiping the composition onto the surface. In one method described herein, it is not necessary for the method to include any steps in addition to simply applying the composition to the surface. Thus, a method that consists essentially of or consists of applying the composition to the surface is described.

It will be appreciated that in order to also provide cleaning it may also be necessary to wipe or scrub the surface. Thus, the invention also provides a method in which the surface is wiped or scrubbed when the composition of the invention is applied to the surface.

The compositions of the invention can be used in the form in which they are provided or can be diluted with water before use. Thus, the invention also provides a method, such as a method as defined above, in which a composition of the invention is diluted before use.

The compositions of the invention that comprise a compound of formula (C) such as N,N-bis(3-aminopropyl)-dodecylamine are particularly useful for controlling or reducing or preventing the formation of fungi.

The anti-microbial compositions of the invention can typically degrade when submersed in water, to provide a rinsate/leachate of low toxicity and which has a short residence time in the environment.

The property of mobility of the product permits materials that are frequently washed or rinsed to be "recharged" with the anti-microbial composition during a routine act of cleaning or maintenance.

Typically, the anti-microbial composition is incorporated into a simple conventional detergent solution or added to a "final rinse" during cleaning. The anti-microbial composition will be drawn, due to the presence of its hydrophobic elements, into the surface of the product to be "recharged". The sanitization properties of the formulation are, therefore, restored without the need for re-manufacture or difficult treatment processes.

Any wash off or rinsates containing the anti-microbial composition or formulation diluted by such a re-charging solution and water would quickly dissociate into the biodegradable components as previously discussed.

According to a further aspect of the invention, there is provided the use of an anti-microbial composition of the invention to reduce or prevent the formation of colonies of microorganisms on a surface at which it is provided.

The anti-microbial compositions of the invention are typically made by a process as described below.
1. The main bulk of the water (approximately 50 to 75% of the total amount of water used) is weighed into suitable vessel.
2. The chelate is added to the water and stirred until homogenous.
3. Optionally, if a polar solvent in addition to water is being used this is added and the mixture is stirred until homogenous.
4. In a separate vessel, a combination of non-ionic surfactant and amphoteric surfactant (if used) and fragrance (if used) is prepared and then added to the mixture obtained in step 4 and the mixture stirred until, homogenous.
5. If any cationic surfactants are used they are added and the mixture stirred until homogenous.
6. The quaternary ammonium compound(s) are added and the mixture stirred until homogenous.
7. The polymer is added and the mixture stirred until homogenous.
8. Water is added to 100% and the mixture stirred.

The person of ordinary skill in the art would know how to adjust the pH of the composition. For example, if this method is being used to produce a high pH composition sodium carbonate and/or sodium bicarbonate and/or other suitable pH adjusters can be added in step 2. If this method is being used to produce a low pH composition, the pH can be adjusted by adding an acid to the composition between steps 7 and 8.

If the composition is to comprise an additional anti-microbial agent such as PHMB, this is added between steps 6 and 7.

The compositions may be prepared in a concentration form (i.e. with little or no polar solvent) and diluted with polar solvent (e.g. water) when used.

### Brief description of the Figures:

Figure 1 shows the results of the cleaning test reported in Example 6.
Figure 2 shows the results of the cleaning test reported in Example 7.

The invention will now be illustrated by the following non-limiting Examples.

### Experimental Residual Efficacy Testing

Aim: Testing was carried out to determine the residual sanitising efficacy of antimicrobial products on non-porous stainless steel surface against certain microorganisms using abrasion steps and microbial challenges over a 24 hour period.

### Equipment

Stainless steel coupons 2cm diameter, grade 304/1.4301 with grade 2B finish (as documented in EN13697:2001)
Tryptone soya agar (TSA), for anti bacterial efficacy testing
Malt Extract Agar (MEA), for anti fungal efficacy testing
Tryptone -saline diluent (as documented in EN1276-2009)
Bovine albumin (fraction V)
Vortex
Validated neutralisation solution
Sterile plastic containers > 3cm and < 5cm in diameter
Forceps
50ml centrifuge tube weighted to -200g
Sterile petri dishes
Inoculating loops
70% (v/v) iso-propanol
Balance to at least 2 decimal places
LAF Class 2 Biologica Safety cabinet
Deionised water
Hard surface polypropylene wipes
Standard Hard water (as referenced in en1276:2009)
Incubator capable of 37°C ± 1 °C

### Method

### Preparation of steel coupons

Steel coupons (stainless steel discs) were rinsed thoroughly with clean hot water (tap water) to remove any contaminating dirt from the surface then rinsed for a few seconds with freshly deionised water. The cleaned coupons were sanitised by submerging in 70% (v/v) iso-propanol for 15 minutes. Using sterile forceps the coupons were transferred to sterile Petri dishes and dried by evaporation under laminar air flow by leaving the lid slightly ajar. Five replicates were used for each time point sampled plus a control set.

### Abrasive Cycle

A 50ml centrifuge tube was weighted so that it weighed approximately 200g (+/- 20g). A professional care wipe was folded twice (4 layers) and wrapped over the lid of the centrifuge tube ensuring a smooth cover. An abrasive cycle consisted of a dry wipe followed by a bacterial challenge and a wet wipe followed by a bacterial challenge (as detailed below).

### a) Wet Wipe

This constituted an abrasion with a polypropylene hard surface wipe wrapped over the lid of a 50 ml centrifuge tube. The wipe was dampened by spraying a mist of standard sterile hard water, using a trigger spray (2 sprays, approximately 50 cm distance from the wipe). The tube was wiped in a forward and backward motion across the stainless steel coupon taking 2-3 seconds, allowing all the downward pressure to be provided by the weight of the centrifuge tube on the coupon surface. The wiped discs were then allowed to dry before the dry wipe cycle was carried out.

### b) Dry Wipe

Mechanism as in the wet wipe cycle however the wipe was not wetted.

### c) Bacterial challenge

The discs were then subject to a bacterial challenge using either a 10⁶ CFU/ml (intermediate time/ wear points) or 10⁸ CFU/ml (0 and final wear cycles) concentration of the required bacterial suspension with an interferring substance. (See below for bacterial challenge preparation).

### Bacterial Challenge Preparation

A bacterial suspension was prepared fresh from a secondary subculture on TSA (subcultures prepared as documented in EN13697:2001). The bacterial suspension was diluted as necessary using tryptone saline, to give a cell concentration of approximately 10⁸ CFU/ml suspension. The concentration of the bacterial suspension was determined using a colorimeter. The suspension was mixed 1:1 with bovine albumin soluion (0.6%) before use to achive a final concentration of 0.3% bovine albumin solution in the bacterial suspension. The 10⁸ CFU/ml concentration of bacterial suspension was used for the initial and final bacterial challenges in the testing.

For re-innoculations (included in the abrasion steps) the 10⁸ CFU/ml suspension was further diluted to 10⁶ CFU/ ml using tryptone-saline solution. The 10⁶ CFU/ml suspension was mixed 1:1 with bovine albumin soluion (0.6%) before use to achive a final concentration of 0.3% bovine albumin solution in the bacterial suspension.

### Test Method (All testing performed using 5 replicates)

1. Time 0hr: 100µl of the product being tested or sterile hard water (for control coupons) was pipetted onto sterile coupons, ensuring the entire surface was covered by spreading the product with an inoculating loop. The coupon was left to dry on a level surface in a biological safety cabinet until completely dry at ambient temperature (21-23°C).
2. (0 wears) A control steel disc was assessed for initial antibacterial efficacy - 10 µL of the bacterial suspension (10⁸ CFU/ml) containing 0.3% bovine albumin was added to the steel disc and after 5 minutes contact time, using sterile tweezers, the disc was placed into a vial containing 10ml neutraliser solution. The vial was shaken briefly to allow mixing, then left to stand for 5 minutes, after which time the vial was vortexed for 30 seconds. Serial dilutions (10⁻⁴) were then prepared in the neutralisation solution and plated out. Molten TSA was poured into the plates and allowed to set before incubation at 37°C for 48 hours.
3. Abrasion samples
   1^{st} abrasive wear cycle - Following the complete desiccation of the 100 µl of product (at least 3 hours) the steel coupons were subjected to their first abrasion wear cycle. This consisted of a dry wipe followed by a bacterial challenge and a wet wipe followed by a bacterial challenge using a 10⁶ CFU/ ml concentration of the bacterial suspension (0.3% bovine albumin concentration). 10 µl of the 10⁶ CFU/ ml bacterial/ bovine albumin suspension was applied to the centre of the disc and gently spread using a sterile disposable loop. The discs were left to dry in a Petri dish in a safety cabinet. Once dried the lids were replaced and the discs left to stand until the next abrasive cycle was required.
   2^{nd} abrasive wear cycle - exactly as above
   3^{rd} abrasive wear cycle (24 hours). A dry wipe followed by a re-inoculation was performed before a final wet wipe was carried out on the discs and the antibacterial efficacy was determined as in the initial activity (0 wears) using the 10⁸ CFU/ml bovine albumin bacterial suspension as follows.

10 µl of the 10⁸ CFU/ml bacterial/bovine albumin suspension was added to the discs and after 5 minutes contact time, using sterile tweezers, the discs were placed into separate vials containing 10ml neutraliser solution. The vials shaken briefly to allow mixing, then left to stand for 5 minutes, after which time the vials were vortexed for 30 seconds. Serial dilutions to (10⁻⁴) were then prepared in the neutralisation solution and plated out. Molten TSA was poured into the plates and allowed to set before incubation at 37°C for 48 hours.

### Neutraliser Validation:

A fresh batch of bacterial suspension was prepared (10⁸ CFU/ml) in tryptone-saline solution from a secondary culture on TSA. The suspension was mixed 1:1 with 0.6% bovine albumin solutions to achieve a final concentration of 0.3% bovine albumin solution in the bacterial suspension.

For each product under test, 100 µl of product was pipette into a sterile container with 10 ml of neutraliser and gently mixed, left for 5 minutes. After the neutralisation the disc was inoculated with 10 µl of 10⁸ CFU/ml of the antimicrobial test suspension and transferred to a vial and mixed well, the vial was allowed to stand for 5 minutes and vortexed for 30 seconds. Serial dilutions to 10⁻⁴ were prepared in the neutraliser solution and plated out. Molten TSA (Tryptone Soya Agar) was poured into the plate and allowed to set before being incubated at 37°C for 48 hours. TVC's (total viable counts of the bacteria grown in the agar) were obtained from the products and log 10 bacterial reductions calculated from discs treated with hard water as a control.

The compositions tested were considered to pass the test and provide a residual antimicrobial benefit if at the end of this test they gave a log 3 or greater bacterial reduction.

### EXAMPLE 1: Simple formulations - Quats/ Chelate/ Surfactant - With and Without Polymer (Mirapol®)

| Project Sharp 12 | Quats/No Mirapol/Chelates/Surfactants | Quats/Mirapol/Chelates/Surfactants |
|---|---|---|
| Lab Code | **RSH009/24E** | **RSH009/24A** |
| Ref | **Sharp 12.E** | **Sharp 12.A** |
| Quats | 4500 | 4500 |
| Mirapol Surf S110 | 0 | 1000 |
| Surfac EDTA Na4 Liquor | 800 | 800 |
| Surfac UN90 90 C9-11 Alcohol Ethoxylate | 1000 | 1000 |
| Lactic Acid | 1760 | 380 |
| pH | 4.05 | 4.05 |
| Formulation comments | Clear | Clear |
| Microbiological results Pseudo (745-12) | 1.65 (0.29) | Not available |
| Microbiological results E.coli (749-12) | 2.88 (0.50) | 20 (0.42) |

These results show that a formulation that does not contain a polymer (Mirapol® Surf S110) does not provide residual antimicrobial performance against either Pseudomonas or E.coli. These results also show that a formulation containing all the essential ingredients of the compositions of the invention provide residual antimicrobial performance.

### Experimental

### Quat Blend Concentrate - RSH008/100A

In a suitably sized vessel Deionised Water (760.00g) was added, followed by the addition of DDQ (Acticide® DDQ40) (200.00g) the mixture was left to stir for 10 minutes on a magnetic. The BAC (Acticide® BAC50M) (40.00g) was then added and left to stir for a further 30 minutes.

### Preparation of 12E

In a suitably sized vessel Deionised water (90.00g) was added, followed by the addition of Tetrasodium EDTA (Surfac® Na4 EDTA Liquor - Surfachem) (0.2g) the mixture was left to stir
on a magnetic stirrer for 15 minutes. The C9-11 Alcohol Ethoxylate (Surfac UN9090 - Surfachem) (0.11g) was then added the mixture was left to stir for a further 15 minutes. The Quat blend (RSH008/100A - Byotrol) (7.00g) was then added and left to stir for a further 10 minutes. The pH was then checked and adjusted to approximately pH 4 using 88% Lactic Acid Sol (Fisher) (0.1g). Deionised water was then added to make sample up to 100g (4.61g) and then stirrer for a further 15 minutes.

### EXAMPLE 2: Low pH mixed surfactant formulations

### Residual performance against Pseudomonas

| Low pH Simple Formulation (pH 4) | No Surfactant | All 4 Key ingredients | No Mirapol | No Chelate, No Mirapol |
|---|---|---|---|---|
| Project Sharp 3 | Quats/Mirapol/Chelates/No Surfactants | Quats/Mirapol/Chelates/Surfactants | Quats/No Mirapol/Chelates/Surfactants | Quats/No Mirapol/No Chelates/Surfactants |
| | TJM006/91A | TJM006/91B | TJM006/91C | TJM006/91E |
| Ref | **Sharp 3.A** | **Sharp 3.B** | **Sharp 3.C** | **Sharp 3.E** |
| Quats | 7000 | 7000 | 7000 | 7000 |
| Mirapol Surf S110 | 2000 | 2000 | 0 | 0 |
| Dissolvine GL47 (GLDA) | 940 | 940 | 940 | 0 |
| Surfac UN90 90 C9-11 Alcohol Ethoxylate | 0 | 4500 | 4500 | 4500 |
| Euroxide DO40 C10 Amine Oxide | 0 | 4000 | 4000 | 4000 |
| Lactic Acid | 880 | 3640 | 4050 | 2550 |
| pH | 4.07 | 4.09 | 4.07 | 4.02 |
| Micro results (Pseudo) (741-12) | >4.88 (0.44) | >4.88 (0.82) | 2.32 (0.49) | 1.75 (0.28) |

These results show that compositions that do not comprise the polymer do not provide the desired level of residual anti-microbial performance. It is known that the addition of high levels of surfactant relative to the amount of quaternary ammonium compound can have an impact on the antimicrobial performance of a composition. The results of Sharp 3E show this effect, however, the addition of component (ii) the polymer and the chelate (v) provides a formulation that has a desirable level of residual antimicrobial performance (> 3 log).

### Experimental for Sharp Series: Sharp 3B

In a suitably sized vessel Deionised Water approx. (78.35g) was added, followed by the addition of Dissolvine GL-47 (Brenntag) (0.2g) the mixture was then left to stir for 10 minutes on a magnetic stirrer. The Alcohol Ethoxylate (Surfac® UN9090 - Surfachem) (0.50g) and C10 Amine Oxide (Euroxide® D40 - EOC) (1.0g) were then added and stirred for a further 15 minutes. After this the Quat blend (4:1 blend of DDAC and BAC) (14.0g) was added and the mixture stirred for a further 10 minutes. The Mirapol® Surf S110 (Rhodia) (0.95g) was then added and stirred for a further 10 minutes. The pH was then checked and adjusted to approximately pH 4 using 8.8% Lactic Acid Sol (Fisher) (4.14g). Deionised water (0.86g) was then added to 100g and stirred for a further 15 minutes.

Samples Sharp 3A, C and E were made using the same procedure as for Sharp 3B but with the omission of the ingredients specified in the table.

### EXAMPLE 3: Low pH mixed surfactant formulations with various chelates - Residual performance against Pseudomonas

| Project Sharp 4 | Quats/Mirapol/Chelates/Surfactants | Quats/Mirapol/Chelates/Surfactants | Quats/Mirapol/Chelates/Surfactants | Quats/Mirapol/Chelates/Surfactants | Quats/Mirapol/Chelates/Surfactants |
|---|---|---|---|---|---|
| | EDTA | EDDS | MGDA | Na Gluconate | GLDA |
| Lab Code | RSH008/98A | R5H008/988 | RSH008/98C | RSH008/98D | TJM006/91B |
| Ref | **Sharp 4.A** | **Sharp 4.B** | **Sharp 4.C** | **Sharp 4.D** | **Sharp 3.B** |
| Quats | 7000 | 7000 | 7000 | 7000 | 7000 |
| Mirapol Surf S110 | 2000 | 2000 | 2000 | 2000 | 2000 |
| Chelate | 1600 | 1500 | 1600 | 5000 | 940 |
| Surfac UN90 90 C9-11 Alcohol Ethoxylate | 4500 | 4500 | 4500 | 4500 | 4500 |
| Euroxide DQ40 C10 Amine Oxide | 4000 | 4000 | 4000 | 4000 | 4000 |
| Lactic Acid | 4400 | 5280 | 4400 | 3960 | 3640 |
| pH | 4.09 | 4.08 | 4.09 | 4.09 | 4.09 |
| Formulation Comments | Clear | Clear | Clear | Clear | Clear |
| Micro Results (Pseudo) (736-12) | 4.49 (0.71) | >4.88 (0.20) | 4.37 (0.79) | 4.25 (0.74) | >4.88 (0.82) |

### Project Sharp 4: Sharp 4A

In a suitably sized vessel Deionised water (75g) was added, followed by the Alcohol Ethoxylate (Surfac® UN9090 - Surfachem) (0.50g) and C10 Amine Oxide (Euroxide® D40 - EOC) (1.0g) was then added and the mixture stirred for 15 minutes. After this the Quat blend (4:1 blend of DDAC and BAC) (14.0g) was added and the mixture stirred for a further 10 minutes. The Mirapol® Surf S110 (Rhodia) (0.95g) was then added and stirred for a further 10 minutes. The Tetrasodium EDTA (Surfac Na4EDTA Liquor) (0.4g) was then added and the mixture stirrered for a further 15 minutes. The pH was then checked and adjusted to approximately pH 4 using 88% Lactic Acid Sol (Fisher) (0.5g). Deionised water (7.65g) was then added to 100g and stirred for a further 15 minutes.

All other formulations were prepared in the same way as Sharp 4A using the alternative chelate.

### EXAMPLE 4: High pH Formulations - Residual performance against Pseudomonas

| Project Sharp 2 | Quats/Mirapol/Chelates/Surfactants | Quats/Mirapol/No Chelates/Surfactants |
|---|---|---|
| | EDTA | n/a |
| Lab Ref (735-747) | RSH008/96A | RSH08/96F |
| Ref | **Sharp 2.A** | **Sharp 2.F** |
| Quats | 7000 | 7000 |
| Mirapol Surf S110 | 2000 | 2000 |
| Na2CO3 | 3600 | 3600 |
| NaHCO3 | 1000 | 1000 |
| Chelate | 1600 | 0 |
| Surfac UN90 90 C9-11 Alcohol Ethoxylate | 4500 | 4500 |
| Euroxide DO40 C10 Amine Oxide | 8000 | 8000 |
| pH | 9.85 | 9.84 |
| Formulation comments Microbiological results 1 (Pseudo) (735, 747) | Clear >4.57 (0.47) | Slightly Hazy 0.21 (0.08) |

These results show that in addition to improving residual antimicrobial performance, at high pH the chelate can also improve the clarity of the formulation. This is of particular interest in achieving an aesthetically favourable product.

The inventors have found that formulating with polymers of the types described herein can sometime cause clarity issues at higher pH (>4.5), in part due to the chemical nature of the monomers. By selection of the surfactant, the chelate and the amount of these ingredients the clarity of the compositions can be improved.

### Experimental Project Sharp 2: Sharp 2A (RSH008/96A)

In a suitably sized vessel Deionised Water (75g) was added, followed by the addition Sodium Carbonate (Fisher) (0.36g) and Sodium Bicarbonate (Fisher) (0.10g) the mixture was left to stir for 15 minutes on a magnetic stirrer. After this the Alcohol Ethoxylate (Surfac® UN9090 - Surfachem) (0.5g) and C10 Amine Oxide (Euroxide® D40- EOC) (2.00g) were added and left to stir for a further 30 minutes. The Quat Blend (4:1 blend of DDAC:BAC) (14.00g) was then added and stirred for a further 10 minutes. The Mirapol® Surf S110 (Rhodia) (0.95g) was then added and stirred for a further 10 minutes, the solution becomes hazy at this point. The Chelate -Tetrasodium EDTA (Surfac® Na4 EDTA Liquor- Surfachem) (0.4g) was then added and stirred for a further 15 minutes, solution cleared on addition of EDTA. The pH was then checked spec pH 9.50 - 10.00. Deionised water (6.69g) was then added to 100g and mixture was then stirred for a further 15 minutes. Sharp 2F was made in the same way with the omission of chelate.

At high pH there can be stability and clarity issues. The use of a chelate can help improve the clarity of a composition and its subsequent stability. The absence of a chelate (EDTA) can have a negative effect on the microbiological performance.

| Project Sharp 2 | Quats/Mirapol/Chelates/Surfactants | Quats/Mirapol/Chelates/Surfactants | Quats/Mirapol/Chelates/Surfactants | Quats/Mirapol/Chelates/Surfactants |
|---|---|---|---|---|
| | EDTA | GLDA | EDTA | GLDA |
| Lab Ref (735-747) | RSH008/96A | RSH008/96E | RSH009/26C | RSH009/26D |
| Ref | **Sharp 2.A** | **Sharp 2.E** | **Sharp 2.G** | **Sharp 2.H** |
| Quats | 7000 | 7000 | 7000 | 7000 |
| Mirapol Surf S110 | 2000 | 2000 | 2000 | 2000 |
| IPA | 0 | 0 | 5976 | 5976 |
| Dowanol | 0 | 0 | 5000 | 5000 |
| Na2CO3 | 3600 | 3600 | 3600 | 3600 |
| NaHCO3 | 1000 | 1000 | 1000 | 1000 |
| Chelate | 1600 | 1880 | 1600 | 1880 |
| Surfac UN90 90 C9-11 Alcohol Ethoxylate | 4500 | 4500 | 4500 | 4500 |
| Euroxide DO40 C10 Amine Oxide | 8000 | 8000 | 8000 | 8000 |
| pH | 9.85 | 9.76 | 10.02 | 10.07 |
| Formulation comments | Clear | Clear | Clear | Clear |
| Microbiological results 1 (Pseudo) (735, 747) | >4.57 (0.47) | >4.00 (0.84) | >4.57 (0) | 4.16 (0.89) |

These results show that the use of a polar solvent in addition to water can help improve the stability of the composition, especially if the composition contains a fragrance. The inclusion of such a solvent does not have a detrimental effect of residual anti-microbial performance. All of the compositions tested in this Example passed the test for residual antimicrobial benefit.

### Experimental

Compositions Sharp 2E, 2G and 2H were made using the method described for compositions Sharp 2A, with the additional of the solvent being added after the surfactant.

### EXAMPLE 5: High pH formulations - with and without polymer (Mirapol®) and with and without chelates - Residual performance against Pseudomonas

| Project Sharp 11 | Quats/Mirapol/Chelates/Surfactants | Quats/Mirapol/Chelates/No Surfactants | Quats/No Mirapol/Chelates/Surfactants | Quats/Mirapol/No Chelates/Surfactants | Quats/No Mirapol/No Chelates/Surfactants |
|---|---|---|---|---|---|
| Lab Code | RSH009/26A | RS009/72B | RSH009/72C | RSH009/72D | RSH009/72E |
| Ref | **Sharp 2A** | **Sharp 11B** | **Sharp 11C** | **Sharp 11D** | **Sharp 11E** |
| Quats | 7000 | 7000 | 7000 | 7000 | 7000 |
| Mirapol Surf S110 | 2000 | 2000 | 0 | 2000 | 0 |
| Na2CO3 | 3600 | 3600 | 3600 | 3600 | 3600 |
| NaHCO3 | 1000 | 1000 | 1000 | 1000 | 1000 |
| EDTA | 1600 | 1600 | 1600 | 0 | 0 |
| Surfac UN90 90 C9-11 Alcohol Ethoxylate | 4500 | 0 | 4500 | 4500 | 4500 |
| Euroxide DO40 C10 Amine Oxide | 8000 | 0 | 8000 | 8000 | 8000 |
| Lactic Acid | 0 | 180 | 1800 | 0 | 2000 |
| pH | 10 | 9.96 | 9.96 | 9.9 | 9.79 |
| Microbiological results Pseudo (747, 760) | >4.57 (0.47) | 4.77 (0) | 0.59 (0.13) | 0.66 (0.14) | 0.56 (0.09) |

These results show that the absence of a chelate and/or a polymer can reduce residual anti-microbial efficacy. They also show that compositions that contain both a polymer and a chelate pass the test for residual antimicrobial benefit. Formulation Sharp 11B contained no surfactants and as a result was an opaque solution, this is not desirable. Although desirable residual performance was achieved the samples that did not contain a surfactant would not be desirable due to poor cleaning performance. Formulation Sharp 2A shows a composition comprising all 4 essential ingredients required by the present invention has desirable residual antimicrobial performance and desirable cleaning ability (see Example 6).

### Experimental

The compositions were prepared as described above for the Sharp 2 series.

### EXAMPLE 6: Comparison of the cleaning properties of high and low pH formulations with and without surfactants.

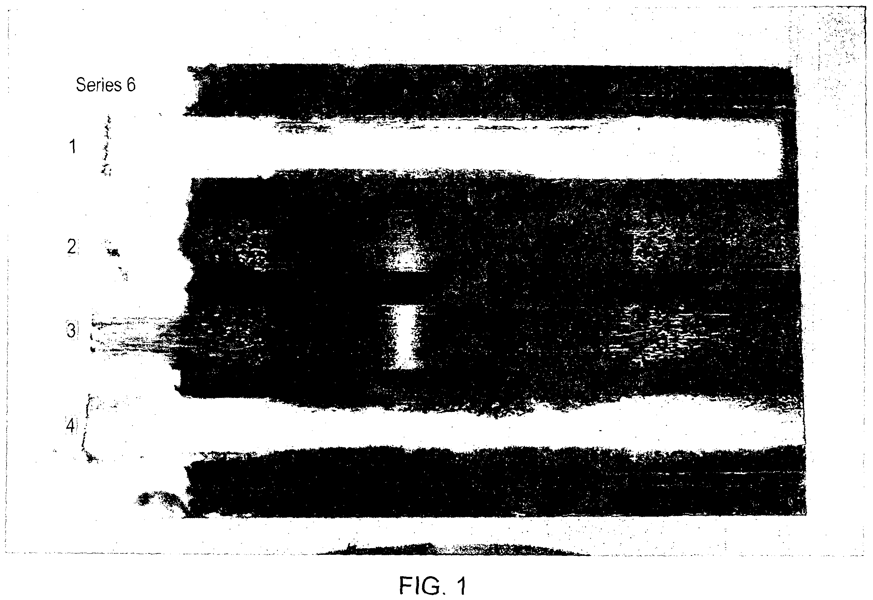

The results of this experiment are shown in Figure 1.
Line 1 - Sharp 2A - High pH composition comprising surfactant
Line 2 - Sharp 11B - High pH composition, no surfactant
Line 3 - Sharp 3A - Low pH composition, no surfactant,
Line 4 - Sharp 3B - Low pH composition comprising surfactant

The results show that surfactants are essential for cleaning and that high pH composition may provide slightly better cleaning than low pH compositions. Mixed surfactants may provide improved cleaning performance, particularly against a range of soils.

### Procedure for Cleaning Tests (Use of Wet Abrasion Scrub Tester Re 903PG)

A white acrylic panel dosed with a composition of simulated bath soil (a combination of calcium carbonate, soap and grease) was loaded onto the Wet Abrasion Scrub Tester with the soiled side facing up. 4 sponges labelled 1-4, were soaked in cold water then squeezed out between 2 tiles. For each product 2 grams was dosed onto a sponge, recording which product was on which sponge. The sponges were the loaded into the Wet Abrasion Scrub Tester in sponge holders 1 to 4. 300 grams of extra weight was added to each sponge holder. The machine was set to 100 hundred wear cycles, and then started. Once the run was complete, the panel was removed from the machine and the level of soil removal assessed on tracks 1 to 4. The tracks were then marked between 0 and 10. 0 corresponds to no soil removal, 10 corresponds to complete soil removal.

### Series 6: High pH with and without Surfactants

| High pH | Quats/ Mirapol/ Chelates/ Surfactants | Quats/ Mirapol/ Chelates/ No Surfactants |
|---|---|---|
| Lab Code | RSH009/26A | RS009/72B |
| Ref | **Sharp 2A** | **Sharp 11B** |
| Quats | 7000 | 7000 |
| Mirapol Surf S110 | 2000 | 2000 |
| Na2CO3 | 3600 | 3600 |
| NaHCO3 | 1000 | 1000 |
| EDTA | 1600 | 1600 |
| Surfac UN90 90 C9-11 Alcohol Ethoxylate | 4500 | 0 |
| Euroxide DO40 C10 Amine Oxide | 8000 | 0 |
| Lactic Acid | 0 | 180/0 |
| pH | 10 | 9.96 |
| Cleaning Capabilities | 9 | 1 |

### Series 6: Low pH with and without Surfactants

| Low pH | Quats/ Mirapol/ Chelates/ No Surfactants | Quats/ Mirapol/ Chelates/ Surfactants |
|---|---|---|
| | TJM006/91A | TJM006/91B |
| Ref | **Sharp 3.A** | **Sharp 3.B** |
| Quats | 7000 | 7000 |
| Mirapol Surf S110 | 2000 | 2000 |
| Dissolvine GL47 (GLDA) | 940 | 940 |
| Surfac UN90 90 C9-11 Alcohol Ethoxylate | 0 | 4500 |
| Euroxide DO40 C10 Amine Oxide | 0 | 4000 |
| Lactic Acid | 880 | 3640 |
| pH | 4.07 | 4.09 |
| Cleaning Results | 2 | 7 |

### Example 7: Cleaning performance

| High pH Simple Formulation - Surfactant Dose Response | | | | |
|---|---|---|---|---|
| Sharp 8 - Series 3 cleaning | Quats/ Mirapol/ No Chelates/No Surfactants | Quats/ Mirapol/ Chelates/No Surfactants | Quats/No Mirapol/ No Chelates/No Surfactants | Quats/ Mirapol/ Chelates/ Surfactants |
| Lab Code | RSH009/10A | RSH009/10B | RSH009/10C | RSH009/10D |
| Ref | **Sharp 8.A** | **Sharp 8.B** | **Sharp 8.C** | **Sharp 8.D** |
| Quats | 7000 | 7000 | 7000 | 7000 |
| Mirapol Surf S110 | 2000 | 2000 | 0 | 2000 |
| Surfac UN90 90 | 0 | 0 | 0 | 4500 |
| Euroxide DO40 | 0 | 0 | 0 | 4000 |
| Dissolvine GL47 (GLDA) | 0 | 940 | 0 | 940 |
| Lactic Acid | 0 | 2640 | 440 | 5280 |
| pH | 3.52 | 3.59 | 3.46 | 3.6 |
| Formulation comments Cleaning Capabilities-Bath Soil | Clear 2 | Cloudy 4 | Clear 2 | Hazy 8 |

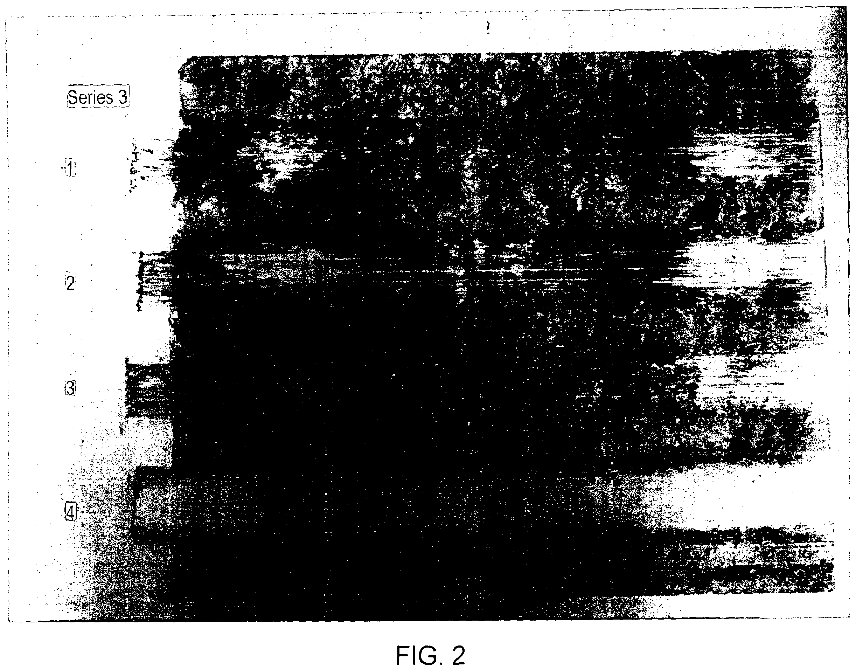

The results of this experiment are shown in Figure 2.
Line 1 - Sharp 8A - Low pH composition, comprising polymer, no surfactant, no chelate
Line 2 - Sharp 8B - Low pH composition, comprising polymer and chelate, no surfactant
Line 3 - Sharp 8C - Low pH composition, no surfactant, no polymer, no chelate
Line 4 - Sharp 8D - Low pH composition comprising surfactant, chelate and polymer

The results show that surfactants facilitate cleaning.

### Experimental: Sharp 8D

In a suitably sized vessel Deionised Water (85g) was added, followed by the addition of Quat Blend (RSH008/100A- Byotrol) (7.0g) the mixture was then left to stir for 10 minutes on a magnetic stirrer. After this the Mirapol® Surf S110 (Rhodia) (0.95g) was added and left to stir for a further 10 minutes. The Dissolvine GL-47(Brenntag) (0.20g) was then added and left to stir for a further 10 minutes. The C10 Amine Oxide (Euroxide® D40- EOC) (1.0g) and C9-11 Alcohol Ethoxylate (Surfac® UN90 90 - Surfachem) (0.5g) were then added and the mixture stirred for a further 15 minutes.

The pH was then checked and adjusted to approximately pH 3.5 using 88% Lactic Acid Sol (Fisher) (0.60g). Deionised water (5.75g) was then added to 100g and the mixture stirred for a further 15 minutes.

Compositions Sharp 8A, B and C were made using the same procedure but with the omission of the indicated ingredients.

## Claims

1. An anti-microbial composition which, when subjected to a three wear cycle test on a non-porous stainless steel, glass or plastics substrate, provides a 3 log or greater reduction in micro-organisms over a 24-hour period and comprises (i) an anti-microbial component comprising a quaternary ammonium component (a) comprising at least one quaternary ammonium compound of formula (A) 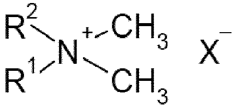 wherein R¹ and R² are each independently a straight chain, unsubstituted and uninterrupted C₈₋₁₂ alkyl group and X⁻ is chloride, bromide, fluoride, iodide, sulphonate, saccharinate, carbonate or bicarbonate and at least one benzalkonium compound of formula (B) 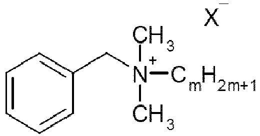 wherein m is from 8 to 18 and X⁻ is chloride, bromide, fluoride, iodide, sulphonate, saccharinate, carbonate or bicarbonate or the benzalkonium compound benzethonium chloride; (ii) a hydrophilic polymer (iii) a polar solvent; (iv) at least one non-ionic surfactant and (v) a chelate; wherein the polymer comprises at least two of the following types of monomer:
(1) a N,N,N-trimethyl-3-((2-methyl-1-oxo-2-propenyl)amino)-1-propanaminium halide monomer or a diallyldimethylammonium halide monomer or a mixture thereof;
(2) an acidic monomer selected from acrylic acid and/or methylacrylic acid; and
(3) a neutral monomer selected from 2-(Dimethylamino)ethyl methacrylate (DMAEMA), N-vinyl pyrrolidone (NVP), N-vinylimidazole, acrylamide, or methacrylamide or a mixture thereof;
and wherein the composition has a pH of 4 or more.

2. A composition according to claim 1 wherein the hydrophilic polymer comprises monomer units selected from 2-(Dimethylamino)ethyl methacrylate (DMAEMA), methacrylamidopropyltrimethyl ammonium chloride (MAPTAC), and methylacrylic acid and mixtures thereof.

3. A composition according to claim 1 wherein the hydrophilic polymer comprises diallyldimethylammonium chloride (DADMAC) and acrylic acid.

4. A composition according to any one of the preceding claims, wherein the hydrophilic polymer is a copolymer containing two or three of the monomer units as defined in claim 1.

5. A composition according to any one of the preceding claims, wherein the benzalkonium compound of formula (B) is benzyldimethyl-n-tetradecyl-ammonium chloride, benzyldimethyl-n-dodecyl-ammonium chloride, benzyl-C₁₂-C₁₆-alkyl-dimethyl-ammonium chloride, or benzyl-cocoalkyl-dimethyl-ammonium chloride and/or the compound of formula (A) is di-n-decyldimethyl ammonium chloride, octyl decyl dimethyl ammonium chloride or dioctyl dimethyl ammonium chloride.

6. A composition according to any one of the preceding claims, wherein the chelate is selected from EDTA (Ethylenediaminetetraacetic acid), Gluconate, GLDA (Glutamic acid diacetic acid), EDDS (Ethylenediamine-N,N'-disuccinic acid), DPTA (Diethylene triamine pentaacetic acid), HEDTA (Hydroxyethyl-ethylenediamine triacetic acid), MGDA (Methyl glycine diacetic acid), PDTA (1,3-propylenediaminetetraacetic acid), and EDG (Ethanoldiglycineic acid) and mixtures thereof.

7. A composition according to any one of the preceding claims, wherein the non-ionic surfactant is an alcohol alkyloxylate or an amine oxide or a mixture thereof.

8. A composition according to any one of the preceding claims having a pH of pH 4.5 or above.

9. A composition according to any one of the preceding claims comprising from 2000 to 10000 ppm of component (i) and/or from 500 to 3000 ppm of hydrophilic polymer; and/or from 400 to 3000 ppm of chelate; and/or from 2000 to 13500 ppm of non-ionic surfactant.

10. A composition according to any one of the preceding claims, which contains less than 3% by weight alcohol.

11. A composition according to any one of the preceding claims, comprising an additional antimicrobial component (b).

12. A composition according to claim 11, wherein the additional anti-microbial agent (b) is selected from polymeric and non-polymeric biguanidines, silver, octenidine HCl, amphoteric compounds, iodophores, phenolic compounds, isothiazalones, nitro bromopropanes, nitrogen based heterocyclic compounds, alkyl betaines, alkyl amine oxides, arginine-based cationic surfactants, anionic amino acid based surfactants and amine anti-microbial agents and mixtures thereof.

13. A composition according to claim 12, wherein the additional anti-microbial agent is polyhexamethylene biguanidine, or a compound of formula (C) 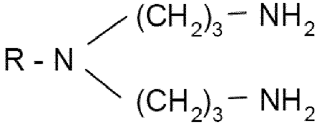 where R is unsubstituted C₈-C₁₈ alkyl.

14. A composition according to claim 13, wherein the compound of formula (C) is N,N bis(3-aminopropyl)-dodecylamine.

15. A composition according to any one of the preceding claims which contains 0.5% by weight or less of formic acid.

16. A composition according to any one of the preceding claims, which is also a cleaning composition.

17. A composition according to claim 1 comprising
(i) (C₁₀H₂₁)₂(CH₃)₂N⁺Cl⁻ and a benzalkonium chloride in a weight ratio of 1:10 to 10:1;
(ii) a polymer comprising DADMAC and acrylic acid;
(iii) water;
(iv) a non-ionic surfact and which is an alcohol ethoxylate or amine oxide or a mixture thereof;
(v) a chelate which is EDTA or GLDA;
and having a pH of at least 2.5.

18. A composition according to any one of the preceding claims for use on hard surfaces.

19. A method of cleaning a hard surface and providing a residual antimicrobial effect to that hard surface, which method comprises applying to the hard surface an antimicrobial composition according to any one of the preceding claims.

20. The use of an anti-microbial composition according to any one of claims 1 to 18 to substantially reduce or control the formation of microbial colonies on or at a hard surface.

21. A method of substantially reducing or controlling the formation of microbial colonies on or at a hard surface, which method comprises applying to that surface an anti-microbial composition according to any one of claims 1 to 18.

22. A method of disrupting, preventing or reducing the adhesion and/or attachment of micro-organisms to a hard surface, which method comprises applying to that surface an anti-microbial composition according to any one of claims 1 to 18.

## Patentansprüche

1. Antimikrobielle Zusammensetzung, die, wenn sie einer Prüfung mit drei Verschleißzyklen an einem Substrat aus nicht porösem rostfreien Stahl, Glas oder Kunststoff unterzogen wurde, eine Reduzierung von Mikroorganismen über einen 24-Stunden-Zeitraum von mindestens 3 log bereitstellt und Folgendes umfasst: (i) eine antimikrobielle Komponente, umfassend eine quartäre Ammoniumkomponente (a), umfassend wenigstens eine quartäre Ammoniumverbindung von Formel (A) 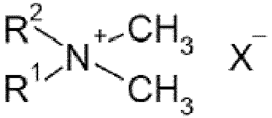 wobei R¹ und R² jeweils unabhängig eine geradkettige, unsubstituierte und ununterbrochene C₈-₁₂-Alkylgruppe sind und X⁻ Chlorid, Bromid, Fluorid, lodid, Sulfonat, Saccharinat, Carbonat oder Bicarbonat ist, und wenigstens eine Benzalkoniumverbindung von Formel (B) 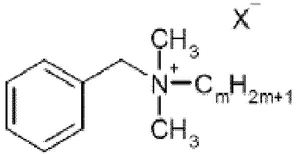 wobei m 8 bis 18 beträgt und X⁻ Chlorid, Bromid, Fluorid, lodid, Sulfonat, Saccharinat, Carbonat oder Bicarbonat oder die Benzalkoniumverbindung Benzethoniumchlorid ist; (ii) ein hydrophiles Polymer; (iii) ein polares Lösungsmittel; (iv) wenigstens ein nichtionisches Tensid und (v) ein Chelat; wobei das Polymer wenigstens zwei der folgenden Monomerarten umfasst:
(1) ein N,N,N-Trimethyl-3-((2-methyl-1-oxo-2-propenyl)amino)-1-propanaminiumhalidmonomer oder ein Diallyldimethylammoniumhalidmonomer oder ein Gemisch daraus;
(2) ein saures Monomer, ausgewählt aus Acrylsäure und/oder Methylacrylsäure; und
(3) ein neutrales Monomer, ausgewählt aus 2-(Dimethylamino)ethylmethacrylat (DMAEMA), N-Vinylpyrrolidon (NVP), N-Vinylimidazol, Acrylamid oder Methacrylamid oder einem Gemisch daraus;
und wobei die Zusammensetzung einen pH-Wert von wenigstens 4 aufweist.

2. Zusammensetzung nach Anspruch 1, wobei das hydrophile Polymer Monomereinheiten umfasst, die ausgewählt sind aus 2-(Dimethylamino)ethylmethacrylat (DMAEMA), Methacrylamidopropyltrimethylammoniumchlorid (MAPTAC) und Methacrylsäure und Gemischen daraus.

3. Zusammensetzung nach Anspruch 1, wobei das hydrophile Polymer Diallyldimethylammoniumchlorid (DADMAC) und Acrylsäure umfasst.

4. Zusammensetzung nach einem der vorhergehenden Ansprüche, wobei das hydrophile Polymer ein Copolymer ist, das zwei oder drei der Monomereinheiten nach Anspruch 1 enthält.

5. Zusammensetzung nach einem der vorhergehenden Ansprüche, wobei die Benzalkoniumverbindung von Formel (B) Benzyldimethyl-n-tetradecylammoniumchlorid, Benzyldimethyl-n-dodecylammoniumchlorid, Benzyl-C₁₂-C₁₆-alkyldimethylammoniumchlorid oder Benzylcocoalkyldimethylammoniumchlorid ist und/oder die Verbindung von Formel (A) Din-decyldimethylammoniumchlorid, Octyldecyldimethylammoniumchlorid oder Dioctyldimethylammoniumchlorid ist.

6. Zusammensetzung nach einem der vorhergehenden Ansprüche, wobei das Chelat ausgewählt ist aus EDTA (Ethylendiamintetraessigsäure), Gluconat, GLDA (Glutaminsäurediessigsäure), EDDS (Ethylendiamin-N,N'-dibernsteinsäure), DPTA (Diethylentriaminpentaessigsäure), HEDTA (Hydroxyethylethylendiamintriessigsäure), MGDA (Methylglycindiessigsäure), PDTA (1,3-Propylendiamintetraessigsäure) und EDG (Ethanoldiglycinsäure) und Gemischen daraus.

7. Zusammensetzung nach einem der vorhergehenden Ansprüche, wobei das nichtionische Tensid ein Alkoholalkyloxylat oder ein Aminoxid oder ein Gemisch daraus ist.

8. Zusammensetzung nach einem der vorhergehenden Ansprüche mit einem pH-Wert von mindestens 4,5.

9. Zusammensetzung nach einem der vorhergehenden Ansprüche, umfassend 2.000 bis 10.000 ppm von Komponente (i) und/oder 500 bis 3.000 ppm hydrophiles Polymer; und/oder 400 bis 3.000 ppm Chelat; und/oder 2.000 bis 13.500 ppm nichtionisches Tensid.

10. Zusammensetzung nach einem der vorhergehenden Ansprüche, die weniger als 3 Gew.-% Alkohol enthält.

11. Zusammensetzung nach einem der vorhergehenden Ansprüche, eine zusätzliche antimikrobielle Komponente (b) umfassend.

12. Zusammensetzung nach Anspruch 11, wobei das zusätzliche antimikrobielle Mittel (b) ausgewählt ist aus polymeren und nichtpolymeren Biguanidinen, Silber, Octenidin-HCl, amphoteren Verbindungen, Iodophoren, Phenolverbindungen, Isothiazolonen, Nitrobrompropanen, heterocyclischen Verbindungen auf Stickstoffbasis, Alkylbetainen, Alkylaminoxiden, kationischen Tensiden auf Argininbasis, Tensiden auf Basis einer anionischen Aminosäure und antimikrobiellen Aminmitteln und Gemischen daraus.

13. Zusammensetzung nach Anspruch 12, wobei das zusätzliche antimikrobielle Mittel Polyhexamethylenbiguanidin oder eine Verbindung von Formel (C) ist 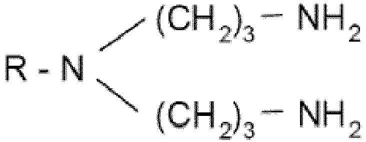 wobei R unsubstituiertes C₈-C₁₈-Alkyl ist.

14. Zusammensetzung nach Anspruch 13, wobei die Verbindung von Formel (C) N,N-Bis(3-aminopropyl)-dodecylamin ist.

15. Zusammensetzung nach einem der vorhergehenden Ansprüche, die höchstens 0,5 Gew.-% Methansäure enthält.

16. Zusammensetzung nach einem der vorhergehenden Ansprüche, die auch eine Reinigungszusammensetzung ist.

17. Zusammensetzung nach Anspruch 1, Folgendes umfassend:
(i) (C₁₀H₂₁)₂(CH₃)₂N⁺Cl⁻ und ein Benzalkoniumchlorid in einem Gewichtsverhältnis von 1:10 bis 10:1;
(ii) ein Polymer, das DADMAC und Acrylsäure umfasst;
(iii) Wasser;
(iv) ein nichtionisches Tensid, das ein Alkoholethoxylat oder ein Aminoxid oder ein Gemisch daraus ist;
(v) ein Chelat, das EDTA oder GLDA ist;
und einen pH-Wert von wenigstens 2,5 aufweisend.

18. Zusammensetzung nach einem der vorhergehenden Ansprüche zur Verwendung auf harten Oberflächen.

19. Verfahren zum Reinigen einer harten Oberfläche und Bereitstellen einer antimikrobiellen Restwirkung auf diese harte Oberfläche, wobei das Verfahren das Auftragen einer antimikrobiellen Zusammensetzung nach einem der vorhergehenden Ansprüche auf die harte Oberfläche umfasst.

20. Verwendung einer antimikrobiellen Zusammensetzung nach einem der Ansprüche 1 bis 18, um die Bildung von mikrobiellen Kolonien auf oder an einer harten Oberfläche im Wesentlichen zu verringern oder zu kontrollieren.

21. Verfahren zum wesentlichen Verringern oder Kontrollieren der Bildung von mikrobiellen Kolonien auf oder an einer harten Oberfläche, wobei das Verfahren das Auftragen einer antimikrobiellen Zusammensetzung nach einem der Ansprüche 1 bis 18 auf diese Oberfläche umfasst.

22. Verfahren zum Stören, Verhindern oder Verringern der Haftung und/oder Anlagerung von Mikroorganismen an eine harte Oberfläche, wobei das Verfahren das Auftragen einer antimikrobiellen Zusammensetzung nach einem der Ansprüche 1 bis 18 auf diese Oberfläche umfasst.

## Revendications

1. Composition antimicrobienne qui, lorsqu'elle est soumise à un test de trois cycles d'usure sur un substrat en acier inoxydable, en verre ou en plastique non poreux, permet une réduction de 3 log ou supérieure dans des micro-organismes pendant une période de 24 heures et comprend (i) un composant antimicrobien comprenant un composant ammonium quaternaire (a) comprenant au moins un composé ammonium quaternaire de la formule (A) 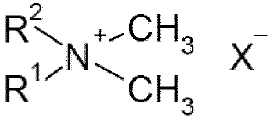 dans laquelle R¹ et R² représentent chacun indépendamment un groupe alkyle en C₈₋₁₂ à chaîne linéaire, non substitué et non interrompu et X⁻ est du chlorure, du bromure, du fluorure, de l'iodure, du sulfonate, du saccharinate, du carbonate ou du bicarbonate et au moins un composé benzalkonium de la formule (B) 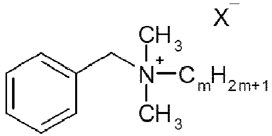 dans laquelle m représente un nombre entre 8 et 18 et X⁻ représente du chlorure, du bromure, du fluorure, de l'iodure, du sulfonate, du saccharinate, du carbonate ou du bicarbonate ou le chlorure de benzéthonium du composé benzalkonium ; (ii) un polymère hydrophile ; (iii) un solvant polaire ; (iv) au moins un tensioactif non ionique et (v) un chélate ; le polymère comprenant au moins deux des types suivants de monomère :
(1) un monomère halogénure de N,N,N-triméthyl-3-((2-méthyl-1-oxo-2-propényl)amino)-1-propanaminium ou un monomère halogénure de diallyldiméthylammonium ou un mélange de ceux-ci ;
(2) un monomère acide choisi parmi un acide acrylique et/ou un acide méthylacrylique ; et
(3) un monomère neutre choisi parmi le méthacrylate de 2-(diméthylamino)éthyle (DMAEMA), le N-vinylpyrrolidone (NVP), le N-vinylimidazole, l'acrylamide ou le méthacrylamide ou un mélange de ceux-ci ;
et la composition ayant un pH de 4 ou supérieur.

2. Composition selon la revendication 1 dans laquelle le polymère hydrophile comprend des unités monomères choisies parmi le méthacrylate de 2-(diméthylamino)éthyle (DMAEMA), le chlorure de méthacrylamidopropyltriméthylammonium (MAPTAC), l'acide méthylacrylique et leurs mélanges.

3. Composition selon la revendication 1 dans laquelle le polymère hydrophile comprend du chlorure de diallyldiméthylammonium (DADMAC) et de l'acide acrylique.

4. Composition selon l'une quelconque des revendications précédentes, dans laquelle le polymère hydrophile est un copolymère contenant deux ou trois des unités monomères comme cela est défini dans la revendication 1.

5. Composition selon l'une quelconque des revendications précédentes, dans laquelle le composé benzalkonium de la formule (B) est du chlorure de benzyldiméthyl-n-tétradécyl-ammonium, du chlorure de benzyldiméthyl-n-dodécyl-ammonium, du chlorure de benzyl-C₁₂-C₁₆-alkyl-diméthyl-ammonium ou du chlorure de benzyl-cocoalkyl-diméthyl-ammonium et/ou le composé de la formule (A) est du chlorure de di-n-décyldiméthylammonium, du chlorure d'octyl-décyl-diméthyl-ammonium ou du chlorure de dioctyl-diméthyl-ammonium.

6. Composition selon l'une quelconque des revendications précédentes, dans laquelle le chélate est choisi parmi l'EDTA (acide éthylènediaminetétraacétique), le gluconate, le GLDA (acide glutamique diacétique), l'EDDS (acide éthylènediamine-N,N'-disuccinique), le DPTA (acide diéthylènetriaminepentaacétique), l'HEDTA (acide hydroxyéthyl-éthylènediamine triacétique), le MGDA (acide méthylglycinediacétique), le PDTA (acide 1,3-propylènediaminetétraacétique) et l'EDG (acide éthanoldiglycinéique) et leurs mélanges.

7. Composition selon l'une quelconque des revendications précédentes, dans laquelle le tensioactif non ionique est un alcoxylate d'alcool, un oxyde d'amine ou un mélange de ceux-ci.

8. Composition selon l'une quelconque des revendications précédentes ayant un pH de 4,5 ou supérieur.

9. Composition selon l'une quelconque des revendications précédentes comprenant de 2 000 à 10 000 ppm de composant (i) et/ou de 500 à 3 000 ppm de polymère hydrophile ; et/ou de 400 à 3 000 ppm de chélate ; et/ou de 2 000 à 13 500 ppm de tensioactif non ionique.

10. Composition selon l'une quelconque des revendications précédentes, qui contient moins de 3 % en poids d'alcool.

11. Composition selon l'une quelconque des revendications précédentes, comprenant un composant antimicrobien (b) supplémentaire.

12. Composition selon la revendication 11, dans laquelle l'agent antimicrobien (b) supplémentaire est choisi parmi les biguanidines polymères et non polymères, l'argent, l'HCl d'octénidine , les composés amphotères, les iodophores, les composés phénoliques, les isothiazolones, les nitrobromopropanes, les composés hétérocycliques à base d'azote, les alkyl-bétaïnes, les oxydes d'alkylamine, les tensioactifs cationiques à base d'arginine, les tensioactifs à base d'acides aminés anioniques et les agents antimicrobiens d'amine et leurs mélanges.

13. Composition selon la revendication 12, dans laquelle l'agent antimicrobien supplémentaire est la polyhexaméthylène biguanidine, ou un composé de la formule (C) 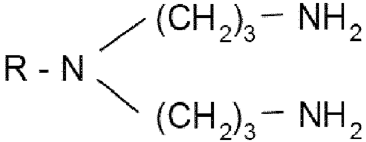 dans laquelle R représente un alkyle en C₈-C₁₈ non substitué.

14. Composition selon la revendication 13, dans laquelle le composé de la formule (C) est la N,N bis(3-aminopropyl)-dodécylamine.

15. Composition selon l'une quelconque des revendications précédentes qui contient au maximum 0,5 % en poids d'acide formique.

16. Composition selon l'une quelconque des revendications précédentes, qui est également une composition de nettoyage.

17. Composition selon la revendication 1 comprenant
(i) du (C₁₀H₂₁)₂(CH₃)₂N⁺Cl⁻ et un chlorure de benzalkonium dans un rapport pondéral de 1:10 à 10:1 ;
(ii) un polymère comprenant du DADMAC et de l'acide acrylique ;
(iii) de l'eau ;
(iv) un tensioactif non ionique et qui est un éthoxylate d'alcool ou un oxyde d'amine ou un mélange de ceux-ci ;
(v) un chélate qui est un EDTA ou un GLDA ;
et ayant un pH d'au moins 2,5.

18. Composition selon l'une quelconque des revendications précédentes à utiliser sur des surfaces dures.

19. Procédé de nettoyage d'une surface dure et d'application d'un effet antimicrobien résiduel sur cette surface dure, ledit procédé comprenant l'application sur la surface dure d'une composition antimicrobienne selon l'une quelconque des revendications précédentes.

20. Utilisation d'une composition antimicrobienne selon l'une quelconque des revendications 1 à 18 pour réduire ou réguler sensiblement la formation de colonies microbiennes sur une surface dure ou au niveau de celle-ci.

21. Procédé pour réduire ou réguler sensiblement la formation de colonies microbiennes sur une surface dure ou au niveau de celle-ci, ledit procédé comprenant l'application sur cette surface d'une composition antimicrobienne selon l'une quelconque des revendications 1 à 18.

22. Procédé de perturbation, de prévention ou de réduction de l'adhésion et/ou de la fixation de microorganismes sur une surface dure, ledit procédé comprenant l'application sur cette surface d'une composition antimicrobienne selon l'une quelconque des revendications 1 à 18.
